(19) Europäisches Patentamt
European Patent Office
Office européen des brevets

(11) EP 0 350 948 B1

(12) EUROPEAN PATENT SPECIFICATION

(45) Date of publication and mention of the grant of the patent: **18.03.1998 Bulletin 1998/12**

(21) Application number: **89112955.3**

(22) Date of filing: **14.07.1989**

(51) Int. Cl.[6]: **C07D 487/22**, A61K 31/40, A61K 49/00 // (C07D487/22, 257:00, 209:00, 209:00, 209:00, 209:00)

# (54) Porphyrin derivatives

Porphyrinderivate

Dérivés de la porphyrine

(84) Designated Contracting States:
**CH DE FR GB IT LI**

(30) Priority: **14.07.1988 JP 173835/88**
**12.06.1989 JP 146615/89**

(43) Date of publication of application:
**17.01.1990 Bulletin 1990/03**

(73) Proprietor:
**TOYOHAKKA KOGYO KABUSHIKI KAISHA**
**Asakuchi-gun Okayama-ken (JP)**

(72) Inventors:
- **SAKATA,Isao**
  **Kasaoka-shi, Okayama-ken (JP)**
- **NAKAJIMA, Susumu**
  **Asahikawa-shi, Hokkaido (JP)**
- **Koshimizu, Koichi**
  **Nara-shi, Nara-ken (JP)**
- **Takada, Hiroyuki**
  **Asakuchi-gun, Okayama-ken (JP)**
- **Inui, Hiroshi**
  **Okayama-ken (JP)**

(74) Representative:
**VOSSIUS & PARTNER**
**Postfach 86 07 67**
**81634 München (DE)**

(56) References cited:
**EP-A- 0 118 913** **EP-A- 0 220 686**

- **R.T. MORRISON et al.: "Organic Chemistry", second edition, 1966, page 1117, Allyn and Bacon Inc.**
- **CHEMICAL ABSTRACTS, vol. 75, 1971, page 12, abstract no. 20984a, Columbus, Ohio, U;**
- **A. VAN DER HEIJDEN et al.: "Coupling of L-histidine methyl ester and L-histidinecontaining peptide esters to ferric protoporphyrin IX chloride", & J. CHEM. SOC. D 1971, (7), 369-70**
- **CHEMICAL ABSTRACTS, vol.91, 1979, page 609, abstract no. 123724a, Columbus, Ohio, US; A.E. VASIL'EV et al.: "Hemin derivatives. VI. General method for the synthesis of hemin amides and esters using water-soluble carbodiimide", &ZH. ORG. KHIM. 1979, 15(4), 828-35**
- **CHEMICAL ABSTRACTS, vol. 112, 1990, pages 712-713, abstract no. 7268h, Columbus, Ohio, US; & JP-A-01 64 481 (TOYO HAKUNI KOGYO CO. LTD) 08-03-1989**

Remarks:
The file contains technical information submitted after the application was filed and not included in this specification

EP 0 350 948 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention concerns a novel composition having affinity to cancerous tissues and containing as an effective component metalloporphyrin derivative(s) for use as a tumor marker, and for diagnosis and treatment of cancers.

It is well known in the art that porphyrin derivatives selectively accumulate in cancer tissues. However, this selectivity is not necessarily sufficient. Porphyrin derivatives, on the other hand, manifest toxicity when exposed to light, and patients to which these substances have been administered are required to stay in the dark for an extended period of time before the porphyrin derivatives which have accumulated in their normal tissues are completely discharged out of their body.

It has been found that metalloporphyrins obtained by introducing a certain metal into the porphyrin skeletons (JP-A-61-83185) and porphyrin derivatives having a chelate-forming group (JP-B-63-13997) had their photo-toxicity decreased while retaining affinity to cancer.

It was we also discovered that amino acid bearing porphyrin derivatives having groups which are labelled by iodine and those which have fluorine (JP-A-64-61481) show similar activities.

When the substance of JP-B-63-13997 was labelled with $^{111}$In or $^{99m}$Tc, and the substance of JP-A-64-61481 with $^{125}$I in order to obtain RI imagings of tumor bearing animals, it was found that the excretion rate of these substances from their liver was quite slow and not necessarily complete, suggesting that these substances are not suitable for diagnosing and treating all kinds of cancers of all the sites.

On the other hand, JP-A-62- 5912, 62-5924, 62-5985 and 62-5986 disclose syntheses of various amino acid derivatives of porphyrin, use of monoamino acid derivatives of chlorin in photo-diagnosis of cancers using a laser fluorescence endoscopy analyser, and cancer treatment with laser. However, said compounds are not metalloporphyrin derivatives, and were developed solely for diagnosis and therapy with laser irradiation. In its table of efficacies, there are noted side effects such as swelling of the legs and damages to the muscles, thus indicating considerable toxicity of said compounds. Use of a metalloporphyrin derivative in diagnosing and treatment of cancer with laser is reported using protoporphyrin Sn and Mg complexes (JP-A-63-264524). These metalloprotoporphyrin derivatives do not accumulate in cancer tissues selectively.

Paramagnetic metals such as Mn and Fe are used as the contrast medium for magnetic resonance imaging (MRI). Philip et al synthesized Mn-tetraphenyl porphine trisulfonate (Mn-TPPS) for use in MRI (Cancer Research 48, 4604 (1988)). This TPPS derivative demonstrates a potent toxicity and lacks selectivity for cancer tissues, and is therefore not fit for practical use.

Sodium Hg-hematoporphyrin and protoporphyrin Co complex were developed and officially registered as pharmaceuticals as the porphyrin compounds with anti-tumor effects. However, the former was judged as "without cause to determine its efficacy" in 1960 and the latter in 1982, and their manufacture and sale were respectively suspended.

As discussed in the foregoing, selectivity and accumulation of a metalloporphyrin compound obtained by introducing a certain metal into the porphyrin skeleton, of a porphyrin derivative having a chelate forming group, and of a tyrosine bearing porphyrin derivative (group labelled by iodine) are not optimal, and therefore diagnosis and treatment of different cancers on all sites are not always possible. There are currently no porphyrin compounds which have a cell killing effect on their own. A need was therefore felt for a metalloporphyrin derivative which demonstrates potent cancer cell destroying effects on its own, or when used with an external energy such as laser, and a metalloporphyrin derivative useful for MRI which is more potent than the prior substances.

There are porphyrin derivatives which are susceptible to photosensitization and others which are not. The former are inherently defective in that phototoxicity appears, but are useful in therapies such as photodynamic therapy (PDT) and that which uses an external energy. On the other hand, the latter are useful as an agent for diagnosing and treating cancer because they do not induce phototoxicity.

EP-A-0 220 686 relates to porphyrin carriers for cancer diagnostic agents which use radioactive metals with a short half-life. The porphyrin carriers use either DTPA or EDTA as a chelating agent to bind the radioactive metallic ions outside of the porphyrin skeleton.

In EP-A-0 118 913 Fe-porphyrin-dipyridoimidazole complexes are disclosed. The compounds disclosed herein have a water-solubility of below 10 mg/ml and thus cannot be administered intravenously.

It is the object of the invention to provide novel substances with affinity to cancer cells which show the desired characteristics for use in treatment and diagnosis of cancer, as a tumor marker and in the missile therapy of cancer.

This object was solved on the basis of the elucidation of physicochemical characteristics of the cell necrosis effect which porphyrin derivatives demonstrate at photoirradiation. There are two stages of destruction; Mechanism I under which the pigments are excited by light to assume the triple state which directly destroys the cancer cells, and Mechanism II under which the pigments in the triple state further excite oxygen to form the singlet state oxygen (activated oxygen) leading to cell necrosis. In the case of porphyrin derivatives, the Mechanism II is considered prevalent. By measuring the triple state life time (the fluorescence and phosphorescence life time) which indicates porphyrin's triple

state, the length of phosphorescence life time is compared to reveal the intensity of photo-sensitivity.
Table 1 shows luminescence properties of various porphyrin derivatives, and indicates that phosphorescence life time is largely affected by the coordination metals of metalloporphyrin derivatives. In other words, phosphorescence life time of metalloporphyrins coordinated with such metals as Zn and Ga is long while that of those coordinated with Mn, Fe, or Cu is very short.

Having considered the above, it was decided to develop metalloporphyrin derivatives with long phosphorescence life time (Ga and Zn complexes) as a drug for treating cancer which uses PDT or an external energy, and those with short phosphorescence life time (Mn, Fe and Cu complexes) as a diagnostic drug for cancer in MRI and RI, and as an antitumor agent on its own. Raving conducted further researches on uses of porphyrin derivatives by noting both characteristics (both long and short phosphorescence life time), it was found that if at least one polyfunctional compound was bonded to the side chain of the metalloporphyrin compound, said porphyrin compound with above mentioned properties would be quickly excreted from the normal tissues while maintaining affinity to cancer.

Table 1

| Compounds | Absorbance (nm) | Fluorescence (nm) | Phosphorescence (nm) | Phosphorescence life time (ms) | |
|---|---|---|---|---|---|
| | | | | Filter paper | Solution |
| PP-Me | 633 | 635 | / | 1.8 | / |
| Mg-PP-Me | 590 | 600 | - | - | - |
| Mn-PP-Me | 570 | 575 | 710 | 20 μs | 20 μs |
| Fe-PP-Me | 645 | - | - | - | - |
| Co-PP-Me | 570 | - | - | - | - |
| Cu-PP-Me | 572 | - | 705 | 20 μs | 100 μs |
| Zn-PP-Me | 582 | 588 | 715 | 35 | / |
| Ga-PP-Me | 570 | 575 | 715 | 50 | 200 |
| In-PP-Me | 583 | 590 | 730 | 7.6 | 14.1 |
| Sn-PP-Me | 583 | 590 | 735 | 7.6 | 13 |
| Me-PPB | 670 | 675 | / | 1.7 | / |
| Photofrin-II | 620 | 625 | 735 | 9 | / |
| PP: protoporphyrin, PPB: pheophorbide | | | | | |

The properties of the present invention porphyrin derivatives do not undergo any substantial change even when bonded to other active substances or combined with external energy.

The subject matter of the present invention are metalloporphyrin compounds expressed by the formula (I)

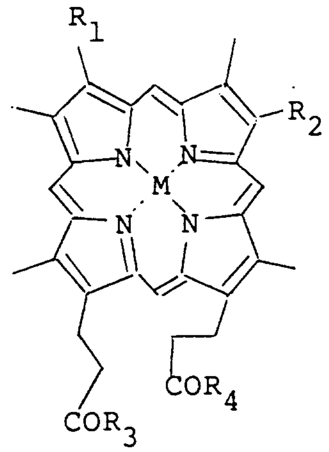

(wherein

$R_1$ and $R_2$ are each -CH(OR)$CH_3$
$R_3$ and $R_4$ are a residue obtained by removing a hydrogen atom from the polyfunctional compound aspartic acid;
R is alkyl, alkenyl or perfluoroalkyl having less than 20 carbon atoms, , a cyclic compound having less than seven members in the ring; and
M is Ga, In, Zn, Mn or Ie

In the above definitions of the symbols, the term "alkyl" means alkyl having usually less than 20 carbon atoms, preferably from 1 to 18 carbon atoms (e.g. methyl, ethyl, n-propyl, hexyl, octyl, decyl, dihydrocitronellyl, undecyl, dodecyl, tetradecyl, and octadecyl), and the term "alkenyl" means alkenyl having less than 20 carbon atoms, preferably from 6 to 16 carbon atoms (e.g. hexenyl, octenyl, geranyl, cytronellyl, and octadecenyl).

The term "perfluoroalkyl" means perfluoroalkyl having less than 20 carbon atoms, preferably from 2 to 11 carbon atoms (e.g. hexafluorobutyl, octafluoropentyl, dodecafluoroheptyl, and pentadecafluorooctyl) and the term "cyclic compound" means cyclic compounds having less than 7 membered rings (e.g. cyclohexyl and menthyl).

Some of these substances are optically active, and levo-rotatory compound, dextro-rotatory compound or DL-rotatory compound may be used or that which has converted to DL-rotatory compound during photosynthesis may be used. They may also be used in the form of salt of alkali metal.

The metalloporphyrin compounds (I) according to the present invention are novel substances,but the compounds per se can be prepared routinely. The process of preparation usually follows the step (a) wherein the porphyrin compound of the formula (I) having $R_1$ and $R_2$ is obtained, the step (b) wherein a metal is introduced therein, and the step (c) wherein thus obtained metalloporphyrin compound is bonded at $R_3$ and $R_4$ with a residue of a polyfunctional compound ie aspartic acid). It is not necessarily important to sequentially conduct the reactions in the order of (a), (b) and (c). The order may be varied as (b), (a) and (c), or (a), (c) and (b).

The steps (a) and (b) can be performed by a routine method such as that disclosed in J. E. Falk: Porphyrins and Metalloporphyrins (Elsevier, 1975). A metalloporphyrin compound having $R_1$ and $R_2$ corresponding to the formula (I) may be prepared according to the methods disclosed in JP-A-61-7279, JP-A-61-83185, and JP-B-63-13997. For the step (a), it is sufficient to introduce R to $R_1$ and $R_2$ side chains of the porphyrin compound (I), and the aspartic acid may be reacted in respect of its hydroxyl group, or with other functional groups (such as $-NH_2$ group). It is preferable to prepare Br derivatives of the porphyrin compound (I) in advance, and to continue reactions with the hydroxyl group of aspartic acid. Other functional groups of the aspartic acid therefore may be protected suitably. For the step (b), a metal chloride , acetate, sulfate or nitrate, is usually used. As metals, Mn, Fe, Zn, Ga, and In are used. The step of metal introduction (b) may be performed before or after the step (a) depending on the need. For instance, the step (b) may be performed first, Br derivatives of metalloporphyrin prepared, and then Br derivative reacted with the functional compound (the step (a)). Instead of artificial synthesis, this compound may be obtained from a natural source such as a plant or an animal.

The thus obtained metalloporphyrin compound is then subjected to the step (c) where it is bonded to aspartic acid. This may be performed by a routine method discussed in Izumiya et al: Basis and Test on Peptide Synthesis (Maruzen, 1985) (in Japanese) and by following a method disclosed in JP-A-64-61481. Instead of artificial synthesis, this compound may be obtained from a natural source such as a plant or an animal.

In this case, since the aspartic acid residue is to be merely introduced into the side chain of the porphyrin compound (I), it may be reacted in respect of its amino group, or with other functional groups (such as -OH group). It is preferable to proceed with the reaction between a carboxyl group at $R_3$ and $R_4$ side chains of the porphyrin compound (I) and the amino group of aspartic acid. Therefore, it is preferable to convert the former carboxyl group and/or the latter amino group to routine reacting groups, or to suitably protect groups which should not participate in the reaction between the two. In either case, reaction-promoting agents such as a dehydrating agent and a deoxidizer or condensing agents may be used.

The process of preparing metalloporphyrin compound (I) is now explained concretely by referring to representative examples. If a metalloporphyrin compound at least either $R_3$ or $R_4$ of which is an OH group ( JP-A-61-7279, JP-A-61-83185, and JP-B-63-13997) is reacted with the amino group-bearing polyfunctional compound aspartic acid in a solvent using a condensing agent (e.g. dicyclohexylcarbodiimide (DCC) or water soluble carbodiimide (WSC)), a metalloporphyrin compound (I) having an aspartic acid side chain at $R_3$ and $R_4$ may be obtained obtained. Their concrete examples, and examples of the metalloporphyrin compounds (I) thus produced are discussed below. Compounds (1) (2) (13) (28) (33) (34) (40) (41) (45) (58) (60) (61) are for illustrative purposes only and not covered by the scope of the present invention

(1) Ga-protoporphynyl diaspartic acid (hereinafter referred to as Ga-PP-diAsp);
(2) 2-[1-(2-hydroxyethyloxy)ethyl]-4-ethenyl-Ga-deuteroporphynyl diaspartic acid (hereinafter referred to as monoEG-Ga-DP-diAsp);
(3) 2,4-bis[1-(2-hydroxyethyloxy)ethyl]-Ga-deuteroporphynyl diaspartic acid (hereinafter referred to as EG-Ga-DP-diAsp);
(4) 2,4-bis(1-methoxyethyl)-Ga-deuteroporphynyl diaspartic acid (hereinafter referred to as $C_1$-Ga-DP-diAsp);
(5) 2,4-bis(1-ethoxyethyl)-Ga-deuteroporphynyl diaspartic acid (hereinafter referred to as $C_2$-Ga-DP-diAsp);
(6) 2,4-bis(1-propoxyethyl)-Ga-deuteroporphynyl diaspartic acid (hereinafter-referred to as $C_3$-Ga-DP-diAsp);
(7) 2,4-bis(1-isopropoxyethyl)-Ga-deuteroporphynyl diaspartic acid (hereinafter referred to as $C_3$(iso)-Ga-DP-diAsp);
(8) 2,4-bis(1-butoxyethyl)-Ga-deuteroporphynyl diaspartic acid (hereinafter referred to as $C_4$-Ga-DP-diAsp);
(9) 2,4-bis(1-pentyloxyethyl)-Ga-deuteroporphynyl diaspartic acid (hereinafter referred to as $C_5$-Ga-DP-diAsp);
(10) 2,4-bis(1-hexyloxyethyl)-Ga-deuteroporphynyl diaspartic acid (hereinafter referred to as $C_6$-Ga-DP-diAsp);
(11) 2,4-bis(1-heptyloxyethyl)-Ga-deuteroporphynyl diaspartic acid (hereinafter referred to as $C_7$-Ga-DP-diAsp);
(12) 2,4-bis(1-octyloxyethyl)-Ga-deuteroporphynyl diaspartic acid (hereinafter referred to as $C_8$-Ga-DP-diAsp);
(13) 2-(1-octyloxyethyl)-4-ethenyl-Ga-deuteroporphynyl diaspartic acid (hereinafter referred to as $C_8$(mono)-Ga-DP-diAsp);
(14) 2,4-bis(1-phenethyloxyethyl)-Ga-deuteroporphynyl diaspartic acid (hereinafter referred to as $C_8$(Phenethyl)-Ga-DP-diAsp);
(15) 2,4-bis(1-nonyloxyethyl)-Ga-deuteroporphynyl diaspartic acid (hereinafter referred to as $C_9$-Ga-DP-diAsp);
(16) 2,4-bis(1-decyloxyethyl)-Ga-deuteroporphynyl diaspartic acid (hereinafter referred to as $C_{10}$-Ga-DP-diAsp);
(17) 2,4-bis(1-geranyloxyethyl)-Ga-deuteroporphynyl diaspartic acid (hereinafter referred to as $C_{10}$(Gera)-Ga-DP-diAsp);
(18) 2,4-bis(1-citronellyloxyethyl)-Ga-deuteroporphynyl diaspartic acid (hereinafter referred to as $C_{10}$(Citro)-Ga-DP-diAsp);
(19) 2,4-bis(1-dihydrocitronellyloxyethyl)-Ga-deuteroporphynyl diaspartic acid (hereinafter referred to as $C_{10}$($H_2$Citro)-Ga-DP-diAsp);
(20) 2,4-bis(1-undecyloxyethyl)-Ga-deuteroporphynyl diaspartic acid (hereinafter referred to as $C_{11}$-Ga-DP-diAsp);
(21) 2,4-bis(1-dodecyloxyethyl)-Ga-deuteroporphynyl diglycine (hereinafter referred to as $C_{12}$-Ga-DP-diGly);
(22) 2,4-bis (1-dodecyloxyethyl)-Ga-deuteroporphynyl diaspartic acid (hereinafter referred to as $C_{12}$-Ga-DP-diAsp);
(23) 2,4-bis(1-tetrahydrofurfuryloxyethyl)-Ga-deuteroporphynyl diaspartic acid (hereinafter referred to as $H_4$Fran-Ga-DP-diAsp);
(24) 2,4-bis(1-(tetrahydro-2-pyranmethyloxy)ethyl]-Ga-deuteroporphynyl diaspartic acid (hereinafter referred to as $H_4$Pyran-Ga-DP-diAsp);
(25) 2,4-bis(1-nicotinyloxyethyl)-Ga-deuteroporphynyl diaspartic acid (hereinafter referred to as Nct-Ga-DP-diAsp);
(26) 2,4-bis(1-octafluoropentyloxyethyl)-Ga-deuteroporphynyl diglycine (hereinafter referred to as $C_5$(F16)-Ga-DP-diGly);
(27) 2,4-bis(1-octafluoropentyloxyethyl)-Ga-deuteroporphynyl diaspartic acid (hereinafter referred to as $C_5$(F16)-Ga-DP-diAsp);
(28) Mg-protoporphynyl diaspartic acid (hereinafter referred to as Mg-PP-diAsp);
(29) 2,4-bis(1-hexyloxyethyl)-Mg-deuteroporphynyl diaspartic acid (hereinafter referred to as $C_6$-Mg-DP-diAsp);

(30) 2,4-bis(1-octyloxyethyl)-Mg-deuteroporphynyl diaspartic acid (hereinafter referred to as $C_8$-Mg-DP-diAsp);
(31) 2,4-bis(1-decyloxyethyl)-Mg-deuteroporphynyl diaspartic acid (hereinafter referred to as $C_{10}$-Mg-DP-diAsp);
(32) 2,4-bis(1-dodecyloxyethyl)-Mg-deuteroporphynyl diaspartic acid (hereinafter referred to as $C_{12}$-Mg-DP-diAsp);
(33) 2-[1-(2-hydroxyethyloxy)ethyl]-4-ethenyl-Mn-deuteroporphynyl diaspartic acid (hereinafter referred to as monoEG-Mn-DP-diAsp);
(34) 2,4-bis(1-(2-hydroxyethyloxy)ethyl]-Mn-deuteroporphynyl diaspartic acid (hereinafter referred to as EG-Mn-DP-diAsp);
(35) 2,4-bis(1-pentyloxyethyl)-Mn-deuteroporphynyl diaspartic acid (hereinafter referred to as $C_5$-Mn-DP-diAsp);
(36) 2,4-bis(1-octyloxyethyl)-Mn-deuteroporphynyl diaspartic acid (hereinafter referred to as $C_8$-Mn-DP-diAsp);
(37) 2,4-bis(1-decyloxyethyl)-Mn-deuteroporphynyl diaspartic acid (hereinafter referred to as $C_{10}$-Mn-DP-diAsp);
(38) 2,4-bis(1-decyloxyethyl)-Mn-deuteroporphynyl di[D]aspartic acid (hereinafter referred to as $C_{10}$-Mn-DP-di[D]Asp);
(39) 2,4-bis(1-decyloxyethyl)-Mn-deuteroporphynyl diglycyldiaspartic acid (hereinafter referred to as $C_{10}$-Mn-DP-diGly-diAsp);
(40) 2,4-bis(1-decyloxyethyl)-Mn-deuteroporphynyl dileucyldiaspartic acid (hereinafter referred to as $C_{10}$-Mn-DP-diLeu-diAsp);
(41) 2,4-bis(1-decyloxyethyl)-Mn-deuteroporphynyl diphenylalanyldiaspartic acid (hereinafter referred to as $C_{10}$-Mn-DP-diPhe-diAsp);
(42) 2,4-bis(1-geranyloxyethyl)-Mn-deuteroporphynyl diaspartic acid (hereinafter referred to as $C_{10}$(Gera)-Mn-DP-diAsp);
(43) 2,4-bis(1-citronellyloxyethyl)-Mn-deuteroporphynyl diaspartic acid (hereinafter referred to as $C_{10}$(Citro)-Mn-DP-diAsp);
(44) 2,4-bis(1-dihydrocitronellyloxyethyl)-Mn-deuteroporphynyl diaspartic acid (hereinafter referred to as $C_{10}$($H_2$Citro)-Mn-DP-diAsp);
(45) 2-(1-dihydrocitronellyloxyethyl)-4-ethenyl-Mn-deuteroporphynyl diaspartic acid (hereinafter referred to as $C_{10}$($H_2$Citro,mono)-Mn-DP-diAsp);
(46) 2,4-bis(1-menthyloxyethyl)-Mn-deuteroporphynyl diaspartic acid (hereinafter referred to as $C_{10}$(Menthyl)-Mn-DP-diAsp);
(47) 2,4-bis(1-undecyloxyethyl)-Mn-deuteroporphynyl diaspartic acid (hereinafter referred to as $C_{11}$-Mn-DP-diAsp);
(48) 2,4-bis(1-dodecyloxyethyl)-Mn-deuteroporphynyl diaspartic acid (hereinafter referred to as $C_{12}$-Mn-DP-diAsp);
(49) 2-(1-dodecyloxyethyl)-4-ethenyl-Mn-deuteroporphynyl diaspartic acid (hereinafter referred to as $C_{12}$(mono)-Mn-DP-diAsp);
(50) 2,4-bis(1-tridecyloxyethyl)-Mn-deuteroporphynyl diaspartic acid (hereinafter referred to as $C_{13}$-Mn-DP-diAsp);
(51) 2,4-bis(1-tetradecyloxyethyl)-Mn-deuteroporphynyl diaspartic acid (hereinafter referred to as $C_{14}$-Mn-DP-diAsp);
(52) 2,4-bis(1-farnesyloxyethyl)-Mn-deuteroporphynyl diaspartic acid (hereinafter referred to as $C_{15}$(Farnesyl)-Mn-DP-diAsp);
(53) 2,4-bis(1-hexadecyloxyethyl)-Mn-deuteroporphynyl diaspartic acid (hereinafter referred to as $C_{16}$-Mn-DP-diAsp);
(54) 2,4-bis(1-octadecyloxyethyl)-Mn-deuteroporphynyl diaspartic acid (hereinafter referred to as $C_{18}$(Oleyl)-Mn-DP-diAsp);
(55) 2,4-bis(1-tetrahydrofurfuryloxyethyl)-Mn-deuteroporphynyl diaspartic acid (hereinafter referred to as $H_4$Fran-Mn-DP-diAsp);
(56) 2,4-bis[1-(tetrahydro-2-pyranmethyloxy)ethyl]-Mn-deuteroporphynyl diaspartic acid (hereinafter referred to as $H_4$Pyran-Mn-DP-diAsp);
(57) 2,4-bis(1-nicotynyloxyethyl)-Mn-deuteroporphynyl diaspartic acid (hereinafter referred to as Nct-Mn-DP-diAsp);
(58) 2,4-bis[1-(2-methylthiopropyloxy)ethyl]-Mn-deuteroporphynyl diaspartic acid (hereinafter referred to as Msp-Mn-DP-diAsp);
(59) 2,4-bis(1-octafluoropentyloxyethyl)-Mn-deuteroporphynyl diaspartic acid (hereinafter referred to as $C_5$(F16)-Mn-DP-diAsp);
(60) 2-(1-octafluoropentyloxyethyl)-4-ethenyl-Mn-deuteroporphynyl diaspartic acid (hereinafter referred to as $C_5$(F8)-Mn-DP-diAsp);
(61) 2,4-bis[1-(2-hydroxyethyloxy)ethyl]-Fe-deuteroporphynyl diaspartic acid (hereinafter referred to as EG-Fe-DP-diAsp);
(62) 2,4-bis(1-octyloxyethyl)-Fe-deuteroporphynyl diaspartic acid (hereinafter referred to as $C_8$-Fe-DP-diAsp);
(63) 2,4-bis(1-decyloxyethyl)-Fe-deuteroporphynyl diaspartic acid (hereinafter referred to as $C_{10}$-Fe-DP-diAsp);
(64) 2,4-bis(1-dodecyloxyethyl)-Fe-deuteroporphynyl diaspartic acid (hereinafter referred to as $C_{12}$-Fe-DP-diAsp);

(65) 2-(1-dodecyloxyethyl)-4-ethenyl-Fe-deuteroporphynyl diaspartic acid (hereinafter referred to as $C_{12}$(mono)-Fe-DP-diAsp);

(66) 2,4-bis(1-decyloxyethyl)-Co-deuteroporphynyl diaspartic acid (hereinafter referred to as $C_{10}$-Co-DP-diAsp);

(67) 2,4-bis(1-dodecyloxyethyl)-Co-deuteroporphynyl diaspartic acid (hereinafter referred to as $C_{12}$-Co-DP-diAsp);

(68) 2,4-bis(1-octyloxyethyl)-Cu-deuteroporphynyl diaspartic acid (hereinafter referred to as $C_8$-Cu-DP-diAsp);

(69) 2,4-bis(1-decyloxyethyl)-Cu-deuteroporphynyl diaspartic acid (hereinafter referred to as $C_{10}$-Cu-DP-diAsp);

(70) 2,4-bis(1-dodecyloxyethyl)-Cu-deuteroporphynyl diaspartic acid (hereinafter referred to as $C_{12}$-Cu-DP-diAsp);

(71) 2,4-bis(1-octyloxyethyl)-Zn-deuteroporphynyl diaspartic acid (hereinafter referred to as $C_8$-Zn-DP-diAsp);

(72) 2,4-bis(1-decyloxyethyl)-Zn-deuteroporphynyl diaspartic acid (hereinafter referred to as $C_{10}$-Zn-DP-diAsp);

(73) 2,4-bis(1-dodecyloxyethyl)-Zn-deuteroporphynyl diaspartic acid (hereinafter referred to as $C_{12}$-Zn-DP-diAsp);

(74) 2,4-bis(1-octyloxyethyl)-In-deuteroporphynyl diaspartic acid (hereinafter referred to as $C_8$-In-DP-diAsp);

(75) 2,4-bis(1-decyloxyethyl)-In-deuteroporphynyl diaspartic acid (hereinafter referred to as $C_{10}$-In-DP-diAsp);

(76) 2,4-bis(1-dodecyloxyethyl)-In-deuteroporphynyl diaspartic acid (hereinafter referred to as $C_{12}$-In-DP-diAsp);

The metalloporphyrin compounds (I) of the present invention are particularly characterized in that they have a two aspartic acid residues at the side chain of the porphyrin skeleton, and as a result they exert various physiological and pharmacological properties. An object of the present invention is to provide metalloporphyrin derivatives for diversified uses such as treatment and diagnoses of diseases, particularly cancer, by varying the metal within the porphyrin skeleton. For instance, a metalloporphyrin derivative with a long phosphorescence life time is used as a chemical which reacts intensely to an external energy (such as laser, microwave, electromagnetic wave, supersonic wave, and medium heat), while that with short phosphorescence life time is used singly as a diagnostic agent or an anti-tumor agent. These porphyrin derivatives accumulate selectively in tumor cells and are excreted therefrom very gradually. On the other hand, they are excreted from the normal organs and cells quickly and do not damage such organs and cells. Most porphyrin derivatives react intensely to light. Introduction of a polyfunctional compound residue to the side chain of a metalloporphyrin derivative according to the present invention promotes excretion from the normal tissues, and offers a derivative designed to maximally utilize the manifestation of phototoxicity while leaving intact selective accumulation property in tumor cells, and a derivative designed to maximally control manifestation of phototoxicity. Based on these properties (cancer affinity, non-phototoxicity, tumor cell necrosis effect of phototoxicity combined with an external energy, tumor cell necrosis effect by single use, etc), the present invention porphyrin derivatives are most useful as therapeutic and diagnostic agents for malignant tumors, as a tumor marker and as an agent for use in the missile therapy of cancers.

The compounds of the present invention are now explained in respect of their pharmacological effects and method of preparation by referring to the following examples.

Experiment 1

Laser Irradiation to the extirpated organ (excited fluorescent spectrum)

Golden hamsters (males, 5 per group, bodyweight approximately 150 g) were implanted subcutaneously tumor cells of the nitrosoamine-induced pancreatic cancer. After 14 to 21 days following implantation, animals were intravenously (i.v.) administered the test substance EG-Ga-DP-diGly (10 mg/ml) which had been diluted with phosphate buffer at 25 mg/kg bodyweight. At 24 hours after administration, the organs including the tumor were extirpated, irradiated with $N_2$-pulsed laser ($N_2$, 337 nm, 2 ns, 400 - 1000 nm), and measured of excited fluorescent spectrum. The wavelengths of 600 - 900 nm were studied using the peak wavelength of NADH at 470 nm as a reference. ($N_2$-PLS measurement). Table 2 shows the results obtained. (Tumor to tissue ratio).

Excited fluorescent spectra of the organs extirpated at 24 hours after administration were measured, and the peak wavelength at 600 - 900 nm was calculated by setting the peak wavelength at 470 nm as the reference 1. The results are shown in Table 2. As fluorescence in Mn, Fe and Cu complexes is not sufficiently intense as to enable $N_2$-PLS measurement, the extraction method was applied to these compounds. At 24 hours following administration, the organs were extracted with an organic solvent (such as chloroform-methanol) via acetone powder, and the obtained extract was measured of UV or HPLC to calculate the tumor to tissue ratio.

As is clear from Table 2, these porphyrin related compounds were found to have a remarkable selective affinity to tumor cells.

Table 2

| compound \ tumor/tissue | | cancer/ liver | cancer/ lung | cancer/ kidney | cancer/ serum |
|---|---|---|---|---|---|
| (2) | monoEG-Ga-DP-diAsp | 2.30 | 1.50 | 1.32 | - |
| (3) | EG-Ga-DP-diAsp | 3.00 | 2.87 | 3.45 | - |
| (4) | $C_1$-Ga-DP-diAsp | 1.19 | 0.78 | 2.27 | 2.50 |
| (5) | $C_2$-Ga-DP-diAsp | 0.71 | 1.25 | 1.25 | 1.16 |
| (6) | $C_3$-Ga-DP-diAsp | 2.95 | 3.42 | 14.44 | 5.91 |
| (7) | $C_{3(iso)}$-Ga-DP-diAsp | 2.63 | 2.33 | 3.03 | 1.54 |
| (8) | $C_4$-Ga-DP-diAsp | 3.54 | 4.47 | 7.72 | 2.00 |
| (9) | $C_5$-Ga-DP-diAsp | 4.07 | 3.89 | 6.58 | 0.89 |
| (10) | $C_6$-Ga-DP-diAsp | 5.33 | 5.33 | 6.86 | 0.98 |
| (11) | $C_7$-Ga-DP-diAsp | 2.96 | 2.92 | 4.88 | 0.89 |
| (12) | $C_8$-Ga-DP-diAsp | 1.47 | 2.20 | 2.12 | 0.29 |
| (14) | $C_{8(Phenethyl)}$-Ga-DP-diAsp | 3.79 | 2.89 | 3.79 | 0.72 |
| (15) | $C_9$-Ga-DP-diAsp | 2.07 | 3.40 | 3.54 | 1.12 |
| (16) | $C_{10}$-Ga-DP-diAsp | 11.54 | 23.08 | 16.44 | 2.78 |
| (17) | $C_{10(Gera)}$-Ga-DP-diAsp | 3.33 | 4.88 | 3.82 | 2.19 |
| (18) | $C_{10(Citro)}$-Ga-DP-diAsp | 11.37 | 12.39 | 15.66 | 2.82 |
| (19) | $C_{10(H_2Citro)}$-Ga-DP-diAsp | 9.09 | 11.11 | 11.49 | 3.50 |
| (22) | $C_{12}$-Ga-DP-diAsp | 4.38 | 7.00 | 8.54 | 1.35 |

Experiment 2

In vivo hemorrhagic necrosis effect on the nitrosoamine-induced pancreatic cancer

Cancer bearing hamsters of Experiment 1 were i.v. administered the test substance $C_6$-Ga-DP-diAsp (10) (10 mg/ml) which had been diluted with phosphate buffer at 25 mg/kg bodyweight and 12.5 mg/kg bodyweight respectively. The animals were exsanguinated and autopsied at 24 hours after administration, and observed. The cancer mass of the treatment group developed hemorrhagic necrosis as shown in Figs. 1, 2 and 3. The organs such as the liver, lungs, kidneys of the treated group excluding the tumor and those of the control group (the group which was not administered the test substance) showed no changes.

Anti-tumor effect of the test material was evaluated by the intensity of hemorrhagic necrosis at 24 hours following administration of the test substance in the manner mentioned above and by the pathological study discussed in Experiment 3. The evaluation was based on the four scales of $G_4$, $G_3$, $G_2$ and $G_1$. The result is shown in Table 3.

Table 3

| Compound | Hemorrhagic necrosis effect |
|---|---|
| (2) monoEG-Ga-DP-diAsp | $G_1$ |
| (4) $C_1$-Ga-DP-diAsp | $G_1$ |
| (5) $C_2$-Ga-DP-diAsp | $G_1$ |
| (6) $C_3$-Ga-DP-diAsp | $G_2$ |
| (7) $C_3$(iso)-Ga-DP-diAsp | $G_2$ |
| (8) $C_4$-Ga-DP-diAsp | $G_4$ |
| (9) $C_5$-Ga-DP-diAsp | $G_4$ |
| (10) $C_6$-Ga-DP-diAsp | $G_4$ |
| (11) $C_7$-Ga-DP-diAsp | $G_4$ |
| (12) $C_8$-Ga-DP-diAsp | $G_3$ |
| (14) $C_8$ (Phenethyl)-Ga-DP-diAsp | $G_1$ |
| (15) $C_9$-Ga-DP-diAsp | $G_2$ |
| (16) $C_{10}$-Ga-DP-diAsp | $G_2$ |
| (17) $C_{10}$(Gera)-Ga-DP-diAsp | $G_2$ |
| (18) $C_{10}$(Citro)-Ga-DP-diAsp | $G_2$ |
| (19) $C_{10}$($H_2$Citro)-Ga-DP-diAsp | $G_2$ |
| (22) $C_{12}$-Ga-DP-diAsp | $G_2$ |
| (36) $C_8$-Mn-DP-diAsp | $G_3$ |
| (37) $C_{10}$-Mn-DP-diAsp | $G_3$ |
| (39) $C_{10}$-Mn-DP-diGly-diAsp | $G_2$ |
| (40) $C_{10}$-Mn-DP-diLeu-diAsp | $G_2$ |
| (41) $C_{10}$-Mn-DP-diPhe-diAsp | $G_2$ |
| (42) $C_{10}$(Gera)-Mn-DP-diAsp | $G_3$ |
| (43) $C_{10}$(Citro)-Mn-DP-diAsp | $G_3$ |
| (44) $C_{10}$($H_2$Citro)-Mn-DP-diAsp | $G_4$ |
| (45) $C_{10}$($H_2$Citro,mono)-Mn-DP-diAsp | $G_2$ |
| (48) $C_{12}$-Mn-DP-diAsp | $G_4$ |
| (49) $C_{12}$(mono)-Mn-DP-diAsp | $G_2$ |
| (51) $C_{14}$-Mn-DP-diAsp | $G_3$ |
| (55) $H_4$Fran-Mn-DP-diAsp | $G_2$ |
| (57) Nct-Mn-DP-diAsp | $G_2$ |
| (62) $C_8$-Fe-DP-diAsp | $G_3$ |
| (64) $C_{12}$-Fe-DP-diAsp | $G_3$ |
| (66) $C_{10}$-Co-DP-diAsp | $G_1$ |
| (67) $C_{12}$-Co-DP-diAsp | $G_1$ |
| (70) $C_{12}$-Cu-DP-diAsp | $G_1$ |
| (73) $C_{12}$-Zn-DP-diAsp | $G_2$ |

Experiment 3

Pathology

Tumor bearing hamsters of Experiment 1 were treated similarly as in Experiment 2 except that they were administered $C_{12}$-Mn-DP-diAsp (48) instead of $C_6$-Ga-DP-diAsp (10). Organs including the tumor (the treated group and the control group) were fixed with formalin, embedded in paraffin, cut into sections, double stained with hematoxylin-eosin, and observed pathologically.

Figs. 4 and 5 show photographs of the tumor cell necrosis of the treated group. There were many cell necrosis images and necrotic layers which were not present in the non-cancerous organs and the control group.

Experiment 4

In vivo cell growth inhibition effect

Tumor bearing hamsters of Experiment 1 were i.v. administered the test substance $C_{10}$-Mn-DP-diAsp (37) (10 mg/ml) diluted by phosphate buffer at 25 mg/kg bodyweight. The tumor size was measured supracutaneously using slide calipers on an appropriate day following administration.

Method of calculation

$$\text{Volume} = 1/2 \times (\text{long diameter}) \times (\text{short diameter})^2$$

Fig. 6 shows tumor cell growth curves of the groups administered $C_{10}$-Mn-DP-diAsp (37) and $C_{12}$-Mn-DP-diAsp (48), and the control group. As is clear from these curves, both treated groups manifested notable tumor growth inhibitory effect compared to the control group, indicating the test substances had carcinostatic activities.

Experiment 5

In vitro photosensitivity test

Leukemia cells of a mouse (P388) were placed in wells (a plate with 24 wells) at 5 x $10^4$/well, and incubated for 2 hours. The test materials $C_8$-Ga-DP-diAsp (12), $C_{10}$-Mn-DP-diAsp (37) and photofrin II each at concentrations of 1.56, 3.13, 6.25, 12.5, 25, and 50 ng/ml (common ratio : 2) were prepared. The specified amounts of these test solutions were added to the above culture, irradiated at 10,000 lux for 1 hour, incubated for 48 hours, and the cells per one well were counted. A group shielded from light using an aluminium foil during irradiation was provided as control. The index of photosensitivity was obtained by dividing the cell counts of the respective groups with the cell count of an untreated group provided separately (cell growth inhibition ratio (%)).

Fig. 7 shows the cell growth inhibition ratio. As is clear from the figure, the irradiated groups treated with Ga complex showed a higher inhibition ratio compared to the control group, manifesting photosensitivity at 1.0 ng/ml or above. As for the groups treated with Mn complex, there was observed no distinct difference in inhibition ratio between the treated groups and the control group, thus indicating no photosensitivity. The inhibition ratio was observed to elevate at concentration of 12.5 ng/ml or higher for each of the test materials. However, this was attributed to the toxicity of the test material per se rather than to irradiation.

Experiment 6

In vivo irradiation therapy using Yag laser

Tumor bearing hamsters of Experiment 1 were i.v. administered the test materials $C_6$-Ga-DP-diAsp (10) and $C_{10}$ -Ga-DP-diAsp (16) (10 mg/ml) at 12.5 mg/kg bodyweight. At 24 hours after administration, the hair above the tumor site was shaved and irradiation with Yag laser commenced (1064 nm, 5 w, 10 sec x 4). At 24 hours following irradiation, the animals were exsanguinated and autopsied for observation. As is clear from Figs. 8 and 9, the cancer mass of the groups administered the test materials was necrotic, while that of the control group (untreated group) showed no such changes.

It is therefore clear that the present invention substance is effective for a therapy using an external energy such as Yag laser.

Reference Example 1

To 1 g of DCHA salt of Ga-protoporphyrin (hereinafter referred to as DCHA salt of Ga-PP) were added and dissolved 60 ml of chloroform and 10 ml of tetrahydrofuran. To the resultant solution was added 0.5 g of glycine ethyl ester hydrochloride (hereinafter referred to as Gly(OEt) • HCl). The obtained solution was then gradually added with 0.1 g of WSC while stirring, and reacted for 2 hours. After the reaction was completed {confirmed by TLC [chloroform : methanol (4 : 1)]}, the reaction solution was washed with water to recover the unreacted substance. After separation, the chloroform layer was concentrated. The obtained concentrate was recrystallized with ethanol ethyl acetate to obtain 0.12 g of Ga-PP-monoGly(OEt). The yield was 16.4%.

Reference Example 2

To 0.1 g of ethyl ester of 1 was added 1/2 N KOH alcohol until the hydrolysis reaction was completed. After the reaction was completed (confirmed by TLC), 10% citric acid solution was added to adjust pH to 5.5, and the resultant solution was extracted with chloroform and concentrated. The resultant concentrate was recrystallized with methanol ethyl acetate to obtain 0.11 g of Ga-PP-monoGly. The yield was 98.0%.

Example 1 〈not covered by the scope of the present invention〉

To 5 g of DCHA salt of monoEG-Ga-DP were added and dissolved 50 ml of chloroform and 100 ml of acetonitrile. To the resultant solution were added 5 ml of 50% Asp(OMe) • HCl DMF solution, and then gradually 3.2 g of WSC while stirring. The procedure similar to those of Reference Examples 1 and 2 was thereafter followed to obtain 3.8 g of monoEG-Ga-DP-diAsp (2). The yield was 86.5%.

Reference Example 3

To 150 g of Ga-protoporphyrin dimethyl ester (hereinafter referred to as Ga-PP-Me) was added 1.5 liter of 6% hydrobromic acid/acetic acid (hereinafter referred to as HBr/HOAc) . The resultant solution was left standing for 24 hours, concentrated under reduced pressure at below 50°C, and the Br derivatives obtained as a result of concentration was added with ethyleneglycol and left standing for 24 hours. After reaction was completed (confirmed by TLC), the product was washed with water, hydrolyzed and recrystallized (aceton-ethyl acetate) to obtain 90 g of 2,4-bis[1-(2-hydroxyethyloxy)ethyl]-Ga-deuteroporphyrin (hereinafter referred to as EG-Ga-DP). The yield was 50.6%.

Example 2 〈not covered by the scope of the present invention〉

To 25 g of DCHA salt of EG-Ga-DP were added and dissolved 250 ml of chloroform and 500 ml of acetonitrile. To the resultant solution were added 12 g of Asp(OMe) • HCl dissolved in 13 ml of DMF, and then gradually 32.5 ml of 10% WSC chloroform solution while stirring. When the reaction was completed after 70 minutes (confirmed with TLC), the reaction liquid was washed with water, extracted and divided with 4% $NaHCO_3$ solution, and added with 10% citric acid solution to the water layer to adjust pH to 5.5. The pH-adjusted water layer was extracted with chloroform and concentrated under reduced pressure to obtain 6.63 g of methyl ester of EG-Ga-DP-monoAsp. By concentrating the chloroform layer under reduced pressure, 2.3 g of methyl ester of EG-Ga-DP-diAsp (3) was also obtained. Hydrolysis was performed similarly as in Reference Example 2 on 0.1 g each of esters to obtain 70 mg of EG-Ga-DP-monoAsp and 50 mg of EG-Ga-DP-diAsp (3). The yields were 23.6% and 5.2% respectively.

Reference Example 4

The procedure similar to Example 3 was followed using 10 g of Ga-PP-Me and 100 ml of 6% HBr/HOAc, and 50 ml each of methyl cellosolve and ethyl cellosolve (hereinafter respectively referred to as MC and EC) instead of ethylene glycol. There were obtained 2.3 g and 1.63 g of 2,4-bis[1-(2-methoxyethyloxy)ethyl]-Ga-deuteroporphyrin and 2,4-bis[1-(2-ethoxyethyloxy)ethyl]-Ga-deuteroporphyrin (hereinafter respectively referred to as MC-Ga-DP and EC-Ga-DP) . The yields were 28.1% and 12.8% respectively.

Reference Example 5

The procedure similar to Reference Example 3 was followed using 6 g of Ga-PP-Me and 60 ml of 10% HBr/HOAc, and 30 ml each of methyl alcohol, ethyl alcohol, propyl alcohol, isopropyl alcohol, butyl alcohol, pentyl alcohol, hexyl alcohol, heptyl alcohol, octyl alcohol, nonyl alcohol, decyl alcohol and dodecyl alcohol separately instead of ethylene

glycol. After hydrolysis, the first two were recrystallized (methanol - ethyl acetate) and the remaining ten were passed through silica gel column chromatography (ethyl acetate - hexane) and recrystallized to obtain respectively 1.0 g, 0.45 g, 2.7 g, 1.3 g, 2.8 g, 2.0 g, 1.6 g, 4.0 g, 1.4 g, 0.84 g, 0.66 g and 2.0 g of 2,4-bis(1-methoxyethyl)-Ga-deuteroporphyrin, 2,4-bis(1-ethoxyethyl)-Ga-deuteroporphyrin, 2,4-bis(1-propoxyethyl)-Ga-deuteroporphyrin, 2,4-bis(1-isopropoxyethyl)-Ga-deuteroporphyrin, 2,4-bis(1-butoxyethyl)-Ga-deuteroporphyrin, 2,4-bis(1-pentyloxy-ethyl)-Ga-deuteroporphyrin, 2,4-bis(1-hexyloxyethyl)-Ga-deuteroporphyrin, 2,4-bis(1-heptyloxyethyl)-Ga-deuteroporphyrin, 2,4-bis(1-octyloxyethyl)-Ga-deuteroporphyrin, 2,4-bis(1-nonyloxyethyl)-Ga-deuteroporphyrin, 2,4-bis(1-decyloxyethyl)-Ga-deuteroporphyrin, and 2,4-bis(1-dodecyloxyethyl)-Ga-deuteroporphyrin (hereinafter respectively referred to as $C_1$-Ga-DP, $C_2$-Ga-DP, $C_3$-Ga-DP, $C_3$(iso)-Ga-DP, $C_4$-Ga-DP, $C_5$-Ga-DP, $C_6$-Ga-DP, $C_7$-Ga-DP, $C_8$-Ga-DP, $C_9$Ga-DP, $C_{10}$-Ga-DP and $C_{12}$-GA-DP). The yields were respectively 15.2%, 6.6%, 38.0%, 18.4%, 38.1%, 26.4%, 20.4%, 49.9%, 16.4%, 9.8%, 7.5% and 21.3%.

Example 3

To 0.5 g of DCHA salt of $C_1$-Ga-DP obtained in Reference Example 5 were added and dissolved 5 ml of chloroform and 2 ml of acetonitrile. To the resultant solution were added 0.5 g of Asp(OMe) • HCl and then gradually 0.51 g of WSC while stirring. The procedure similar to those of Reference Examples 1 and 2 was thereafter followed to obtain 0.44 g of $C_1$-Ga-DP-diAsp (4). The yield was 100%.

Example 4

To 0.5 g of DCHA salt of $C_2$-Ga-DP obtained in Reference Example 5 were added and dissolved 5 ml of chloroform and 2 ml of acetonitrile. To the resultant solution were added 0.5 g of Asp(OMe) • HCl, and then gradually 0.47 g of WSC while stirring. The procedure similar to those Reference of Examples 1 and 2 was thereafter followed to obtain 0.42 g of $C_2$-Ga-DP-diAsp (5). The yield was 95.2%.

Example 5

To 0.5 g of DCHA salt of $C_3$-Ga-DP obtained in Reference Example 5 were added and dissolved 5 ml of chloroform and 2.5 ml of acetonitrile. To the resultant solution were added 0.75 g of Asp(OMe) • HCl, and then gradually 0.52 g of WSC while stirring. The procedure similar to those of Reference Examples 1 and 2 was thereafter followed to obtain 0.32 g of $C_3$-Ga-DP-diAsp (6). The yield was 72.2%.

Example 6

To 0.2 g of DCHA salt of $C_3$(iso)-Ga-DP obtained in Reference Example 5 were added and dissolved 4.5 ml of chloroform and 1.5 ml of acetonitrile. To the resultant solution were added 0.2 g of Asp(OMe) • HCl, and then gradually 0.31 g of WSC while stirring. The procedure similar to those of Reference Examples 1 and 2 was thereafter followed to obtain 0.11 g of $C_3$(iso)-GA-DP-diAsp (7). The yield was 62.2%.

Example 7

To 0.5 g of DCHA salt of $C_4$-Ga-DP obtained in Reference Example 5 were added and dissolved 5 ml of chloroform and 2.5 ml of acetonitrile. To the resultant solution were added 0.75 g of Asp(OMe) • HCl, and then gradually 0.59 g of WSC while stirring. The procedure similar to those of Reference Examples 1 and 2 was thereafter followed to obtain 0.44 g of $C_4$-Ga-DP-diAsp (8). The yield was 100%.

Example 8

To 0.2 g of DCHA salt of $C_5$-Ga-DP were added and dissolved 4.5 ml of chloroform and 1.5 ml of acetonitrile. To the resultant solution were added 0.2 g of Asp(OMe) • HCl, and then gradually 0.25 g of WSC while stirring. The procedure similar to those of Reference Examples 1 and 2 was thereafter followed to obtain 0.17 g of $C_5$-Ga-DP-diAsp (9). The yield was 95.5%.

Example 9

To 0.75 g of DCHA salt of $C_6$-Ga-DP obtained in Reference Example 5 were added and dissolved 14 ml of chloroform and 7 ml of acetonitrile. To the resultant solution were added 1 g of Asp(OMe) • HCl, and then gradually 0.68 g of

WSC while stirring. The procedure similar to those of Reference Examples 1 and 2 was thereafter followed to obtain 0.5 g of $C_6$-Ga-DP-diAsp (10). The yield was 74.6%.

Example 10

To 0.43 g of DCHA salt of $C_7$-Ga-DP obtained in Reference Example 5 were added and dissolved 4 ml of chloroform and 2 ml of acetonitrile. To the resultant solution were added 0.44 g of Asp(OMe) • HCl, and then gradually 0.59 g of WSC while stirring. The procedure similar to those of Reference Examples 1 and 2 was thereafter followed to obtain 0.34 g of $C_7$-Ga-DP-diAsp (11). The yield was 88.3%.

Example 11

To 1.5 g of DCHA salt of $C_8$-Ga-DP were added and dissolved 80 ml of chloroform and 30 ml of acetonitrile. To the resultant solution were added 2 g of Asp(OMe) • HCl, and then gradually 1 g of WSC while stirring. The procedure similar to those of Reference Examples 1 and 2 was thereafter followed to obtain 0.16 g of $C_8$-Ga-DP-diAsp (12). The yield was 12.7%.

Reference Example 6

The procedure similar to that of Example 3 was followed using 6 g of Ga-PP-Me and 60 ml of 3% HBr/HOAc, and 30 ml of octyl alcohol instead of ethylene glycol. After hydrolysis, the resultant substance was passed through silica gel column chromatography (ethyl acetate - hexane), and recrystallized to obtain 0.98 g of the mixture of $C_8$-Ga-DP and 2-(1-octyloxyethyl)-4-ethenyl-Ga-deuteroporphyrin (hereinafter referred to as $C_8$(mono)-Ga-DP). The yield was 11.7%.

Example 12 〈not covered by the scope of the present invention〉

To 6 g of DCHA salt of the mixture of $C_8$-Ga-DP and $C_8$(mono)-Ga-DP was added and dissolved 120 ml of chloroform. To the resultant solution were added 8 g of Asp(OMe) • HCl, and then gradually 5.5 g of WSC while stirring. The procedure similar to those of Examples 7, 8 and 54 was thereafter followed to obtain 0.3 g of $C_8$(mono)-Ga-diAsp (13). The yield was 6.3%.

Reference Example 7

The procedure similar to Example 3 was followed using 6 g of Ga-DP-Me and 60 ml of 10% HBr/HOAc, and 30 ml of phenethyl alcohol instead of ethylene glycol. After hydrolysis, the resultant substance was passed through silica gel column chromatography (ethyl acetate - hexane) and recrystallized to obtain 2.9 g of $C_8$(phenethyl)-Ga-DP. The yield was 66.1%.

Example 13

To 0.2 g of DCHA salt of $C_8$(phenethyl)-Ga-DP obtained in Reference Example 7 were added and dissolved 4.5 ml of chloroform and 1.5 ml of acetonitrile. To the resultant solution were added 0.2 g of Asp(OMe) • HCl, and then gradually 0.25 g of WSC while stirring. The procedure similar to those of Reference Examples 1 and 2 was thereafter followed to obtain 0.06 g of $C_8$(phenethyl)-Ga-DP-diAsp (14). The yield was 33.5%.

Example 14

To 0.17 g of DCHA salt of $C_9$-Ga-DP obtained in Reference Example 5 were added and dissolved 10 ml of chloroform and 5 ml of acetonitrile. To the resultant solution were added 0.2 g of Asp(OMe) • HCl, and then gradually 0.27 g of WSC while stirring. The procedure similar to those of Reference Examples 1 and 2 was thereafter followed to obtain 0.15 g of $C_9$-Ga-DP-diAsp (15). The yield was 98.0%.

Example 15

To 0.54 g of DCHA salt of $C_{10}$-Ga-DP were added and dissolved 10 ml of chloroform and 5 ml of acetonitrile. To the resultant solution were added 0.54 g of Asp(OMe) • HCl, and then gradually 0.54 g of WSC while stirring. The procedure similar to those of Reference Examples 1 and 2 was thereafter followed to obtain 0.48 g of $C_{10}$-Ga-DP-diAsp (16). The yield was 98.4%.

Reference Example 8

The procedure similar to Example 3 was followed using 4.5 g of Ga-PP-Me and 45 ml of 10% HBr/HOAc, and 25 ml of geraniol instead of ethylene glycol. After hydrolysis, the resultant substance was passed through silica gel column chromatography (ethyl acetate - hexane), and recrystallized to obtain 1.0 g of $C_{10}$(Gera)-Ga-DP. The yield was 15.2%.

Example 16

To 0.5 g of DCHA salt of Reference Example 8 were added and dissolved 10 ml of chloroform and 5 ml of acetonitrile. To the resultant solution were added 0.5 g of Asp(OMe) • HCl, and then gradually 0.73 g of WSC while stirring. The procedure similar to those of Examples 1 and 2 was thereafter followed to obtain 0.42 g of $C_{10}$(Gera)-Ga-DP-diAsp (17). The yield was 93.1%.

Reference Example 9

The procedure similar to those of Reference Examples 3 and 8 was followed using 3 g of Ga-PP-Me and 30 ml of 10% HBr/HOAc, and 18 ml of citronellol instead of ethylene glycol to obtain 1.4 g of $C_{10}$(Citro)-Ga-DP. The yield was 31.8%.

Example 17

To 0.5 g of DCHA salt of Reference Example 9 were added and dissolved 10 ml of chloroform and 5 ml of acetonitrile. To the resultant solution were added 0.5 g of Asp(OMe) • HCl, and then gradually 0.72 g of WSC while stirring. The procedure similar to those of Reference Examples 1 and 2 was thereafter followed to obtain 0.43 g of $C_{10}$(Citro)-Ga-DP-diAsp (18). The yield was 95.3%.

Reference Example 10

The procedure similar to Reference Example 3 was followed using 3 g of Ga-PP-Me and 30 ml of 10% HBr/HOAc, and 18 ml of dihydrocitronellol instead of ethylene glycol to obtain 1.1 g of $C_{10}$($H_2$Citro)-Ga-DP. The yield was 24.8%.

Example 18

To 0.5 g of DCHA salt of Reference Example 10 were added and dissolved 10 ml of chloroform and 5 ml of acetonitrile. To the resultant solution were added 0.5 g of Asp(OMe) • HCl, and then gradually 0.72 g of WSC while stirring. The procedure similar to those of Reference Examples 1 and 2 was thereafter followed to obtain 0.42 g of $C_{10}$($H_2$Citro)-Ga-DP-diAsp (19). The yield was 93.8%.

Example 19

To 0.5 g of DCHA salt of $C_{12}$-Ga-DP were added and dissolved 5 ml of chloroform and 2 ml of acetonitrile. To the resultant solution were added 5 g of Asp(OMe) • HCl, and then gradually 0.35 g of WSC while stirring. The procedure similar to those of Reference Examples 1 and 2 was thereafter followed to obtain 0.44 g of $C_{12}$-Ga-DP-diAsp (22). The yield was 97.1%.

Reference Example 11

The procedure similar to those of Reference Examples 3 and 8 was followed using 4.5 g of Ga-PP-Me and 45 ml of 10% HBr/HOAc, and 25 ml each of tetrahydrofurfuryl alcohol and tetrahydropyran-2-methanol instead of ethylene glycol to obtain 3.3 g of $H_4$Fran-Ga-DP and 3.2 g of $H_4$Pyran-Ga-DP. The yields were 55.9% and 52.4% respectively.

Example 20

To 0.2 g of DCHA salt of $H_4$Fran-Ga-DP obtained in Reference Example 11 were added and dissolved 4 ml of chloroform and 2 ml of acetonitrile. To the resultant solution were added 0.3 g of Asp(OMe) • HCl, and then gradually 0.2 g of WSC while stirring. The procedure similar to those of Examples 7 and 8 was thereafter followed to obtain 0.17 g of $H_4$Fran-Ga-DP-diAsp (23) . The yield was 95.1%.

Example 21

To 0.3 g of DCHA salt of $H_4$Pyran-Ga DP obtained in Reference Example 71 were added and dissolved 6 ml of chloroform and 3 ml of acetonitrile. To the resultant solution were added 0.4 g of Asp(OMe) • HCl, and then gradually 0.3 g of WSC while stirring. The procedure similar to those of Reference Examples 1 and 2 was thereafter followed to obtain 0.25 g of $H_4$Pyran-Ga-DP-diAsp (24). The yield was 92.9%.

Reference Example 12

The procedure similar to those of Examples 3 and 8 was followed using 3 g of PP-Me and 30 ml of 10% HBr/HOAc, and 18 ml each of ethylene glycol, octyl alcohol, decyl alcohol, undecyl alcohol, dodecyl alcohol, tetradecyl alcohol, hexadecyl alcohol and octadecyl alcohol separately to obtain 3.2 g, 1.78 g, 1.0 g, 0.89 g, 1.12 g, 0.75 g, 0.09 g, and 0.8 g of 2,4-bis[1-(2-hydroxyethyloxy)ethyl]deuteroporphyrin, 2,4-bis(1-octyloxyethyl)deuteroporphyrin, 2,4-bis(1-decyloxyethyl)deuteroporphyrin, 2,4-bis(1-undecyloxyethyl)-deuteroporphyrin, 2,4-bis(1-dodecyloxyethyl)-deuteroporphyrin, 2,4-bis(1-tetradecyloxyethyl)-deuteroporphyrin, 2,4-bis(1-hexadecyloxyethyl)deuteroporphyrin, and 2,4-bis(1-octadecyloxyethyl)deuteroporphyrin (hereinafter referred to as EG-DP, $C_8$-DP, $C_{10}$-DP, $C_{11}$-DP, $C_{12}$-DP, $C_{14}$-DP, $C_{16}$-DP, and $C_{18}$-DP respectively). The yields were respectively 88.9%, 42.6%, 22.4%, 19.1%, 23.6%, 14.9%, 1.7% and 14.3%.

Reference Example 13

To 20 ml of acetic acid were added 1 g of PP-Me, 1 g of manganese acetate and 1 g of sodium acetate, suspended, and then reacted for 20 minutes under heating (75 - 78°C). After reaction (confirmed by TLC), the reaction solution was added with water to precipitate crystals. The obtained precipitates were filtered off, dried and then hydrolized as in Reference Example 8 to obtain 0.74 g of Mn-protoporphyrin (hereinafter referred to as Mn-PP). The yield was 70.8%.

Reference Example 14

To 10 g each of 2-[1-(2-hydroxyethyloxy)ethyl]-4-ethenyl-deuteroporphyrin (hereinafter referred to as monoEG-DP) and EG-DP were added and dissolved separately 60 ml of chloroform and 20 ml of methanol. To the resultant substances were respectively added 10.2 g of manganese acetate dissolved in 8 ml of methanol while stirring, heated and reacted at 70 - 75°C. After reaction (confirmed by TLC), the reaction solution was concentrated under reduced pressure. The obtained concentrate was then washed with water, filtered, and the filtrate was dried to obtain 2.2 g of monoEG-Mn-DP and 2.35 g of EG-Mn-DP respectively. The yields were 22.0% and 20.8%.

Example 22 〈not covered by the scope of the present invention〉

To 5 g of DCHA salt of monoEG-Mn-DP of Reference Example 14 were added and dissolved 50 ml of chloroform and 100 ml of acetonitrile. To the resultant solution were added 2.5 g of Asp(OMe) • HCl, and then gradually 3.2 g of WSC while stirring. The procedure similar to those of Reference Examples 1 and 2 was thereafter followed to obtain 1.1 g of monoEG-Mn-DP-diAsp (33). The yield was 27.0%.

Reference Example 15

To 5 g each of $C_8$-DP, $C_{10}$-DP, $C_{11}$-DP, $C_{12}$-DP, $C_{14}$-DP, $C_{16}$-DP and $C_{18}$-DP were added separately and dissolved 30 ml of chloroform and 10 ml of methanol. To the resultant solutions were respectively added 5.1 g of manganese acetate dissolved in 4 ml of methanol, and the products were treated similarly as in Example 82 and Mn ion complexed to obtain respectively 5.71 g, 4.75 g, 4.29 g, 4.56 g, 5.0 g, 5.1 g and 5.5 g of $C_8$-Mn-DP, $C_{10}$-Mn-DP, $C_{11}$-Mn-DP, $C_{12}$-Mn-DP, $C_{14}$-Mn-DP, $C_{16}$-Mn-DP and $C_{18}$-Mn-DP. The yields were respectively 84.3%, 66.4%, 58.4%, 60.5%, 63.1%, 61.4% and 63.4%.

Reference Example 16

To 20 g of Mn-PP-Me was added and dissolved 200 ml of 10% HBr/HOAc. After being left standing for 24 hours, the resultant solution was concentrated under reduced pressure at 50°C or below, and the obtained Br derivative was added and dissolved with 100 ml of octanol. The resultant solution was left standing for 24 hours. After the reaction (confirmed by TLC), the reaction product was washed with water, hydrolized as in Example 8, and recrystallized (ethyl acetate - hexane) to obtain 15 g of $C_8$-Mn-DP. The yield was 55.1%.

Example 23

To 0.7 g of DCHA salt of $C_8$-Mn-DP were added and dissolved 14 ml of chloroform and 7 ml of methanol. To the resultant solution were added 0.93 g of Asp(OMe) • HCl, and then gradually 1.1 g of WSC while stirring. The procedure similar to those of Reference Examples 1 and 2 was thereafter followed to obtain 0.53 g of $C_8$-Mn-DP-diAsp (36). The yield was 90.0%.

Reference Example 17

To 0.5 g of DCHA salt of $C_{10}$-Mn-DP obtained in Reference Example 15 were added and dissolved 11.4 ml of chloroform and 3.8 ml of acetonitrile. To the resultant solution were added 0.6 g of Gly(OEt) • HCl, and then gradually 0.6 g of WSC while stirring. The procedure similar to those of Reference Examples 1 and 2 was thereafter followed to obtain 0.37 g of $C_{10}$-Mn-DP-diGly. The yield was 96.7%.

Reference Example 18

To 0.9 g of DCHA salt of crude $C_{10}$-Mn-DP were added and dissolved 18 ml of chloroform and 8 ml of acetonitrile. To the resultant solution were added 1 g of Leu(OMe) • HCl, and then gradually 1.7 g of WSC while stirring. The procedure similar to those of Reference Examples 1 and 2 was thereafter followed to obtain respectively 0.36 g and 0.01 g of $C_{10}$-Mn-DP-diLeu and $C_{10}$(mono)-Mn-DP-diLeu. The yields were respectively 47.4% and 1.2%.

Example 24

To 0.45 g of DCHA salt of $C_{10}$-Mn-DP were added and dissolved 5 ml of chloroform and 4 ml of acetonitrile. To the resultant solution were added 0.59 g of Asp(OMe) • HCl, and then gradually 7 ml of 10% DCC chloroform solution while stirring. The procedure similar to those of Reference Examples 1, 2 was thereafter followed to obtain 0.1 g of $C_{10}$-Mn-DP-diAsp (37). The yield was 26.2%.

Example 25

To 0.45 g of DCHA salt of $C_{10}$-Mn-DP were added and dissolved 9 ml of chloroform and 4 ml of acetonitrile. To the resultant solution were added 0.59 g of [D]Asp(OMe) • HCl, and then gradually 0.7 g of WSC while cooling and stirring. The procedure similar to those of Reference Examples 1, 2 was thereafter followed to obtain 0.15 g of $C_{10}$-Mn-DP-di[D]Asp (38). The yield was 39.4%.

Reference Example 19

To 0.5 g of DCHA salt of $C_{10}$-Mn-DP were added and dissolved 12 ml of chloroform and 4 ml of acetonitrile. To the resultant solution were added 0.7 g of Phe(OMe) • HCl, and then gradually 0.75 g of WSC while stirring. The procedure similar to those of Reference Examples 1 and 2 was thereafter followed to obtain 0.44 g of $C_{10}$-Mn-DP-diPhe. The yield was 98.6%.

Example 26

To 0.13 g of DCHA salt of $C_{10}$-Mn-DP-diGly obtained in Reference Example 17 were added and dissolved 3 ml of chloroform and 1 ml of acetonitrile. To the resultant solution were added 0.12 g of Asp(OMe) • HCl, and then gradually 0.18 g of WSC while stirring. The procedure similar to those of Reference Examples 1 and 2 was thereafter followed to obtain 0.11 g of $C_{10}$-Mn-DP-diGly-diAsp (39). The yield was 93.2%.

Example 27 〈not covered by the scope of the present invention〉

To 0.33 g of DCHA salt of $C_{10}$-Mn-DP-diLeu obtained in Reference Example 18 were added and dissolved 4 ml of chloroform and 1.5 ml of acetonitrile. To the resultant solution were added 0.34 g of Asp(OMe) • HCl, and then gradually 0.33 g of WSC while stirring. The procedure similar to those of Reference Examples 1 and 2 was thereafter followed to obtain 0.24 g of $C_{10}$-Mn-DP-diLeu-diAsp (40). The yield was 79.5%.

Example 28 (not covered by the scope of the present invention)

To 0.32 g of DCHA salt of $C_{10}$-Mn-DP-diPhe obtained in Reference Example 19 were added and dissolved 7.5 ml of chloroform and 2.5 ml of acetonitrile. To the resultant solution were added 0.3 g of Asp(OMe) • HCl, and then gradually 0.25 g of WSC while stirring. The procedure similar to those of Reference Examples 1 and 2 was thereafter followed to obtain 0.23 g of $C_{10}$-Mn-DP-diPhe-diAsp (41). The yield was 78.3%.

Reference Example 20

The procedure similar to those of Reference Examples 3 and 8 was followed using 1 g of PP-Me and 10 ml of 10% HBr/HOAc, and 6 g each of geraniol, citronellol, dihydrocitronellol and menthol respectively instead of ethylene glycol to obtain 2,4-bis(1-geranyloxyethyl)-deuteroporphyrin, 2,4-bis(1-citronellyloxyethyl)deuteroporphyrin, 2,4-bis(1-dihydrocitronellyloxyethyl)-deuteroporphyrin, and 2,4-bis(1-menthyloxyethyl)-deuteroporphyrin (hereinafter respectively referred to as $C_{10}$(Gera)-DP, $C_{10}$(Citro)-DP, $C_{10}$($H_2$Citro)-DP and $C_{10}$(Menthyl)-DP). Each of the compounds obtained was treated similarly as in Reference Example 15 and Mn ion complexed to respectively obtain 0.88 g, 0.4 g, 0.56 g and 0.08 g of $C_{10}$(Gera)-Mn-DP, $C_{10}$(Citro)-Mn-DP, $C_{10}$($H_2$Citro)-Mn-DP, and $C_{10}$(Menthyl)-Mn-DP. The yields were respectively 49.3%, 22,6%, 31.5% and 4.5%.

Example 29

To 0.5 g of DCHA salt of $C_{10}$(Gera)-Mn-DP obtained in Reference Example 20 were added and dissolved 10 ml of chloroform and 5 ml of acetonitrile. To the resultant solution were added 0.5 g of Asp(OMe) • HCl, and then gradually 0.4 g of WSC while stirring. The procedure similar to those of Reference Examples 1 and 2 was thereafter followed to obtain 0.4 g of $C_{10}$(Gera)-Mn-DP-diAsp (42). The yield was 95.2%.

Example 30

To 0.33 g of DCHA salt of crude mixture of $C_{10}$(Citro)-Mn-DP obtained in Reference Example 20 were added and dissolved 6.6 ml of chloroform and 3.3 ml of acetonitrile. To the resultant solution were added 0.33 g of Asp(OMe) • HCl, and then gradually 0.27 g of WSC while stirring. The procedure similar to those of Reference Examples Reference 1 and 2 was thereafter followed to obtain 0.27 g of Asp derivative mixture. The mixture was then passed through octylsilica (C8) high performance liquid chromatograph [eluent: MeOH-$H_2O$ (19:1)], to obtain 0.04 g and 0.12 g of $C_{10}$(Citro)-Mn-DP-monoAsp and $C_{10}$(Citro)-Mn-DP-diAsp (43) respectively. The yields were 15.8% and 42.9% respectively.

Example 31

To 0.66 g of DCHA salt of crude mixture of $C_{10}$($H_2$Citro)-Mn DP obtained in Reference Example 20 were added and dissolved 13.2 ml of chloroform and 6.6 ml of acetonitrile. To the resultant solution were added 0.66 g of Asp(OMe) • HCl, and then gradually 0.54 g of WSC while stirring. The procedure similar to those of Reference Examples 1 and 2 was thereafter followed to obtain 0.58 g of Asp derivative mixture. The mixture was then passed through octylsilica (C8) high performance liquid chromatograph [eluent: MeOH-$H_2O$ (19:1)], to obtain 0.23 g and 0.04 g of $C_{10}$($H_2$Citro)-Mn-DP-diAsp (44) and $C_{10}$($H_2$Citro,mono)-Mn-DP-diAsp (45) respectively. The yields were 38.3% and 8.2% respectively.

Examples 32 (not covered by the scope of the present invention)

To 0.8 g of DCHA salt of crude $C_{12}$-Mn-DP obtained in Reference Example 15 were added and dissolved 15 ml of chloroform and 5 ml of acetonitrile. To the resultant solution were added 0.8 g of Asp(OMe) • HCl, and then gradually 7 ml of 10% WSC chloroform solution while stirring. The procedure similar to those of Reference Examples 1, 2 and Example 30 was thereafter followed to obtain 0.5 g and 0.15 g of $C_{12}$-Mn-DP-diAsp (48) and $C_{12}$(mono)-Mn-DP-diAsp (49) respectively. The yields were 73.1% and 25.8% respectively.

Examples 33

To 0.6 g of DCHA salt of $C_{14}$-Mn-DP obtained in Reference Example 15 were added and dissolved 15 ml of chloroform and 5 ml of acetonitrile. To the resultant solution were added 2.25 g of Asp(OMe) • HCl, and then gradually 2 g of WSC dissolved in 7 ml of chloroform while stirring. The procedure similar to those of Reference Examples 1 and 2 was thereafter followed to obtain 0.5 g of $C_{14}$-Mn-DP-diAsp (51). The yield was 97.0%.

Reference Example 21

The procedure similar to Reference Examples 3 and 8 was followed using 5 g of PP-Me and 75 ml of 10% HBr/HOAc, and 30 ml each of farnesol, octadecenol, phytol, tetrahydrofurfuryl alcohol, tetrahydropyran-2-methanol, 3-pyridine-methanol and methionol respectively instead of ethylene glycol to obtain 2,4-bis(1-farnesyloxyethyl)deuteroporphyrin, 2,4-bis(1-octadecenyloxyethyl)deuteroporphyrin, 2,4-bis(1-phytyloxyethyl)deuteroporphyrin, 2,4-bis(1-tetrafurfuryloryethyl)deuteroporphyrin, 2,4-bis[1-(tetrahydro-2-pyranmethyloxy)ethyl]-deuteroporphyrin, 2,4-bis(1-nicotinyloxyethyl)-deuteroporphyrin and 2,4-bis[1-(2-methylthiopropyloxy)ethyl]-deuteroporphyrin (hereinafter referred to as $C_{15}$(farnesyl)-DP, $C_{18}$(Oleyl)-DP, $C_{20}$(Phytyl) -DP, $H_4$Fran-DP, $H_4$Pyran-DP, Nct-DP and Msp-DP respectively). Each of the compounds obtained was treated similarly as in Reference Example 15 and Mn ion complexed to respectively obtain 3.52g, 0.54 g, 0.3 g, 2.25 g, 2.84 g, 1.7 g and 1.58 g of $C_{15}$(Farnesyl)-Mn-DP, $C_{18}$(Oleyl)-Mn-DP, $C_{20}$(Phytyl)-Mn-DP, $H_4$Fran-Mn-DP, $H_4$Pyran-Mn-DP, Nct-Mn-DP and Msp-Mn-DP respectively. The yields were 35.2%, 5.0%, 2.7%, 28.3%, 34.6%, 21,1% and 19.7% respectively.

Example 34

To 0.06 g of DCHA salt of $C_{16}$-Mn-DP obtained in Reference Example 15 were added and dissolved 9 ml of chloroform and 3 ml of acetonitrile. To the resultant solution were added 0.5 g of Asp(OMe) • HCl, and then gradually 0.6 g of WSC while stirring. The procedure similar to those of Reference Examples 1 and 2 was thereafter followed to obtain 0.01 g of $C_{16}$-Mn-DP-diAsp (53). The yield was 19.3%.

Example 35

To 0.52 g of DCHA salt of $C_{18}$(Oleyl)-Mn-DP obtained in Reference Example 21 were added and dissolved 10.4 ml of chloroform and 5.2 ml of acetonitrile. To the resultant solution were added 0.52 g of Asp(OMe) • HCl, and then gradually 0.6 g of WSC while stirring. The procedure similar to those of Reference Examples 1 and 2 was thereafter followed to obtain 0.43 g of $C_{18}$(Oleyl)-Mn-DP-diAsp (54). The yield was 95.2%.

Example 36

To 0.32 g of DCHA salt of $H_4$Fran-Mn-DP obtained in Reference Example 21 were added and dissolved 6.6 ml of chloroform and 3.3 ml of acetonitrile. To the resultant solution were added 0.33 g of Asp(OMe) • HCl, and then gradually 0.27 g of WSC while stirring. The procedure similar to those of Reference Examples 1 and 2 was thereafter followed to obtain 0.28 g of $H_4$Fran-Mn-DP-diAsp (55). The yield was 98.4%.

Example 37

To 0.3 g of DCHA salt of $H_4$Pyran-Mn-DP obtained in Reference Example 21 were added and dissolved 6 ml of chloroform and 3 ml of acetonitrile. To the resultant solution were added 0.3 g of Asp(OMe) • HCl, and then gradually 0.25 g of WSC while stirring. The procedure similar to those of Reference Examples 1 and 2 was thereafter followed to obtain 0.24 g of $H_4$Pyran-Mn-DP-diAsp (56). The yield was 83.0%.

Example 38

To 0.32 g of DCHA salt of Nct-Mn-DP obtained in Reference Example 21 were added and dissolved 6.6 ml of chloroform and 3.3 ml of acetonitrile. To the resultant solution were added 0.33 g of Asp(OMe) • HCl, and then gradually 0.27 g of WSC while stirring. The procedure similar to those of Reference Examples 1 and 2 was thereafter followed to obtain 0.17 g of Nct-Mn-DP-diAsp (57). The yield was 63.5%.

Example 39

To 0.52 g of DCHA salt of Msp-Mn-DP obtained in Reference Example 21 were added and dissolved 10.4 ml of chloroform and 5.2 ml of acetonitrile. To the resultant solution were added 0.52 g of Asp(OMe) • HCl, and then gradually 0.6 g of WSC while stirring. The procedure similar to those of Examples 1 and 2 was thereafter followed to obtain 0.4 g of Msp-Mn-DP-diAsp (58). The yield was 92.0%.

Reference Example 22

The procedure similar to those of Reference Examples 3 and 8 was followed using 5 g of PP-Me and 75 ml of 10% HBr/HOAc, and 25 ml of octafluoropentyl alcohol instead of ethylene glycol to obtain 1.6 g of 2,4-bis(1-octafluoropentyloxyethyl)-deuteroporphyrin (hereinafter referred to as $C_5$(F16)-DP). The resultant compound was treated similarly as in Example 90 and Mn ion complexed to obtain 1.68 g of $C_5$(F16)-Mn-DP. The yield was 16.7%.

Example 40

To 0.3 g of DCHA salt of crude mixture of the compound obtained in Reference Example 22 were added and dissolved 6 ml of chloroform and 3 ml of acetonitrile. To the resultant solution were added 0.3 g of Asp(OMe) • HCl, and then gradually 0.3 g of WSC while stirring. The procedure similar to those of Reference Examples 1 and 2 was thereafter followed to obtain 0.33 g of Asp derivative mixture. The mixture was then passed through octylsilica (C8) high performance liquid chromatograph [eluent: MeOH-$H_2O$ (3:1)], to obtain 0.1 g and 0.03 g of $C_5$(F16)-Mn-DP-diAsp (59) and $C_5$(F8)-Mn-DP-diAsp (60) respectively. The yields were 38.6% and 14.0% respectively.

Reference Example 23

To 0.5 g each of $C_8$-DP and $C_{12}$-DP obtained in Example 75 was separately added 10 ml of dimethylacetoamide, and the resultant mixture was suspended with 1.6 g of ferric chloride $6H_2O$, and reacted for 3 hours under heating (100°C). The reaction solution was then concentrated, added with water, and crystals were precipitated. The obtained precipitates were then filtered off, dried and then treated similarly as in Reference Examples 2 and 8 to obtain 0.4 g and 0.14 g of $C_8$-Fe-DP and $C_{12}$-Fe-DP respectively. The yields were 72.2% and 25.6% respectively.

Example 41

To 0.25 g of DCHA salt of $C_8$-Fe-DP obtained in Reference Example 23 were added and dissolved 5 ml of chloroform and 2.5 ml of acetonitrile. To the resultant solution were added 0.25 g of Asp(OMe) • HCl, and then gradually 0.25 g of WSC while stirring. The procedure similar to those of Reference Examples 1 and 2 was thereafter followed to obtain 0.22 g of $C_8$-Fe-DP-diAsp (62). The yield was 98.0%.

Example 42

To 0.12 g of DCHA salt of crude mixture of $C_{12}$-Fe-DP obtained in Reference Example 23 were added and dissolved 4 ml of chloroform and 2 ml of acetonitrile. To the resultant solution were added 0.12 g of Asp(OMe) • HCl, and then gradually 0.27 g of WSC while stirring. The procedure similar to those of Reference Examples 1 and 2 was thereafter followed to obtain 0.09 g of Asp derivative mixture. The resultant mixture was then passed through octylsilica (C8) high performance liquid chromatograph [eluent: MeOH-$H_2O$ (99:1)], to obtain 0.04 g and 0.005 g of $C_{12}$-Fe-DP-diAsp (64) and $C_{12}$(mono)-Fe-DP-diAsp (65) respectively. The yields were respectively 36.7% and 5.6%.

Reference Example 24

To 0.64 g each of $C_{10}$-DP and $C_{12}$-DP obtained in Reference Example 12 were added and dissolved respectively 20 ml of chloroform and 10 ml of methanol. The resultant solutions were further added with 1.82 g of cobalt acetate dissolved in 10 ml of methanol and reacted. The reaction solutions were concentrated and added with water, and crystals were precipitated. The obtained precipitates were filtered off, dried and then treated similarly as in Reference Example 2 and to obtain 0.6 g and 0.53 g of $C_{10}$-Co-DP and $C_{12}$-Co-DP respectively. The yields were 82.9% and 77.9% respectively.

Example 43

To 0.3 g of DCHA salt of $C_{10}$-Co-DP obtained in Reference Example 24 were added and dissolved 15 ml of chloroform and 5 ml of acetonitrile. To the resultant solution were added 0.3 g of Asp(OMe) • HCl, and then gradually 0.35 g of WSC while stirring. The procedure similar to those of Reference Examples 1 and 2 was thereafter followed to obtain 0.26 g of $C_{10}$-Co-DP-diAsp (66). The yield was 96.4%.

Example 44

To 0.43 g of DCHA salt of $C_{12}$-Co-DP obtained in Reference Example 24 were added and dissolved 10 ml of chloroform and 5 ml of acetonitrile. To the resultant solution were added 0.43 g of Asp(OMe) • HCl, and then gradually 0.4 g of WSC while stirring. The procedure similar to those of Reference Examples 1 and 2 was thereafter followed to obtain 0.23 g of $C_{12}$-Co-DP-diAsp (67). The yield was 59.2%.

Reference Example 25

To 0.3 g of $C_{12}$-DP obtained in Reference Example 12 were added and dissolved 10 ml of chloroform and 5 ml of methanol. To the resultant solution was added 0.64 g of copper acetate and reacted. The reaction liquid was concentrated, added with water, and crystals were precipitated. The obtained precipitates were filtered off, dried and then treated similarly as in Reference Examples 2 and 8 to obtain 0.31 g of $C_{12}$-Cu-DP. The yield was 97.0%.

Example 45

To 0.4 g of DCHA salt of $C_{12}$-Cu-DP obtained in Reference Example 25 were added and dissolved 10 ml of chloroform and 3 ml of acetonitrile. To the resultant solution were added 0.4 g of Asp(OMe) • HCl, and then gradually 0.4 g of WSC while stirring. The procedure similar to those of Reference Examples 1 and 2 was thereafter followed to obtain 0.3 g of $C_{12}$-Cu-DP-diAsp (70). The yield was 83.0%.

Reference Example 26

To 0.5 g each of EG-DP and $C_{12}$-DP obtained in Reference Example 12 were respectively added and dissolved 15 ml of chloroform and 2.5 ml of methanol. The resultant solutions were added with 2.2 ml of 5% zinc acetate methanol solution and reacted. The reaction liquids were concentrated and added with water, and crystals were precipitated. The obtained precipitates were filtered off, dried and then treated similarly as in Reference Examples 2 and 8 to obtain 0.41 g and 0.5 g of EG-Zn-DP and $C_{12}$-Zn-DP respectively. The yields were 74.5% and 93.7% respectively.

Example 46

To 0.25 g of DCHA salt of $C_{12}$-Zn-DP obtained in Reference Example 26 were added and dissolved 5 ml of chloroform and 2.5 ml of acetonitrile. To the resultant solution were added 0.25 g of Asp(OMe) • HCl, and then gradually 0.25 g of WSC while stirring. The procedure similar to those of Reference Examples 1 and 2 was thereafter followed to obtain 0.21 g of $C_{12}$-Zn-DP-diAsp (73). The yield was 92.9%.

The porphyrin derivatives according to the present invention have the properties to accumulate in tumor cells, to react to external energy, and to destroy tumor cells. Since they do not manifest toxicity in normal cells, they are most useful as a therapeutic agent or a diagnostic agent for cancer. Fig. 1 is a photograph showing hemorrhagic necrosis of a cancer mass at 24 hours after administration of $C_n$-Ga-DP-diAsp (n = odd numbers, 8, 6, 9, 11 and 15), and Fig. 2 is also a photograph showing hemorrhagic necrosis of a cancer mass at 24 hours after administration of $C_n$-Ga-DP-diAsp (n = even numbers, 5, 8, 10, 12, 16 and 22).

Fig. 3 is also a photograph showing hemorrhagic necrosis of cancer masses at 24 hours after administration of $C_{10}$-Mn-DP-PheAsp on the right, $C_{10}$(Gera)-Mn-DP-diAsp (42) at the center, and $C_8$-Fe-DP-diAsp (62) on the left.

Fig. 4 is a photograph showing necrotic tumor cells of the group administered $C_{12}$-Mn-DP-diAsp (48).

Fig. 5 is a photograph of tumor cells of the control group.

Fig. 6 is a graph showing tumor cell growth curves after administrations of the following substances.

○ : Control group
● : $C_{10}$-Mn-DP-diAsp (37)
■ : $C_{12}$-Mn-DP-diAsp (48)

Fig. 7 is a graph showing cell growth inhibitions after administrations of the following substances.

▲ : $C_8$-Ga-DP-diAsp (12) without irradiation
△ : $C_8$-Ga-DP-diAsp (12) with irradiation
● : $C_{10}$-Mn-DP-diAsp (37) without irradiation
○ : $C_{10}$-Mn-DP-diAsp (37) with irradiation
■ : Photofrin II without irradiation

□ : Photofrin II with irradiation

Fig. 8 is a photograph showing an animal given Yag laser irradiation at 24 hours following administration of $C_6$-Ga-DP-diAsp (10).

Fig. 9 is a photograph showing an animal of the control group given Yag laser irradiation.

Fig. 10 through 19 are graphs showing mass spectra (SIMS-NOBA); Fig. 10 of methyl ester of EG-Ga-DP-monoSer, Fig. 11 EG-Ga-DP-monoAsp, Fig. 12 methyl ester of $C_8$-Ga-DP-diSer, Fig. 13 methyl ester of $C_8$-Ga-DP-diAsp (12), Fig. 14 (12), Fig. 15 methyl ester of $C_8$(mono)-Ga-DP-diAsp (13), Fig. 16 $C_8$-Mn-DP-diAsp (36), Fig. 17 $C_{10}$ -Mn-DP-diAsp (37), Fig. 18 $C_8$-Ga-DP, and Fig. 19 ethyl ester of monoEG-Cu-DP-diGly. Figs. 20 through 24 are graphs showing NMR spectra ($^1$H-NMR); Fig. 20 $C_4$-Ga-DP-diAsp (51), Fig. 21 $C_6$-Ga-DP-diAsp (10), Fig. 22 $C_8$-Ga-DP-diAsp (12), Fig. 23 $C_{10}$($H_2$Citro)-Ga-DP-diAsp (19), and Fig. 24 $H_4$Fran-Ga-DP-diAsp (23).

## Claims

1. Metalloporphyrin compounds represented by the general formula (I)

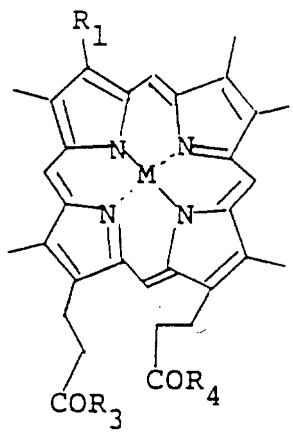

wherein

$R_1$ and $R_2$ are each -CH(OR)$CH_3$
$R_3$ and $R_4$ are a residue obtained by removing a hydrogen atom from an aspartic acid;
R is alkyl, alkenyl or perfluoroalkyl having less than 20 carbon atoms, a cyclic compound having less than seven members in the ring; and
M is Ga, In, Zn, Mn or Fe.

2. A metalloporphyrin compound of the general formula (I) according to claim 1 for use as a therapeutic or diagnostic agent for malignant tumors, as a tumor marker or as an agent for use in the missile therapy of cancers.

## Patentansprüche

1. Metallporphyrinverbindungen der allgemeinen Formel (I)

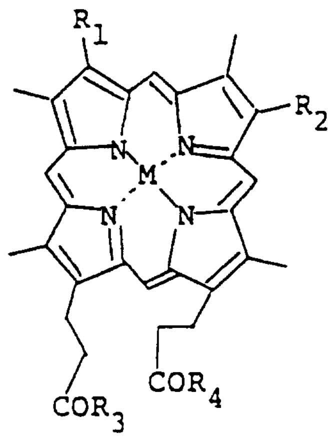

wobei

$R_1$ und $R_2$ jeweils $-CH(OR)CH_3$ bedeuten;
$R_3$ und $R_4$ einen Rest darstellen, der durch Entfernen eines Wasserstoffatoms aus einer Asparaginsäure erhalten wird;
R ein Alkyl-, Alkenyl- oder Perfluoralkylrest mit weniger als 20 Kohlenstoffatomen, oder eine cyclische Verbindung mit weniger als 7 Gliedern im Ring ist; und
M für Ga, In, Zn, Mn oder Fe steht.

2. Metall-porphyrinverbindung der allgemeinen Formel (I) nach Anspruch 1 zur Anwendung als ein therapeutisches oder diagnostisches Mittel für bösartige Tumoren, als ein Tumormarker oder als ein Mittel zur Verwendung in der Strahlentherapie bei Krebserkrankungen.

**Revendications**

1. Composés métalloporphyrine représentés par la formule générale (I)

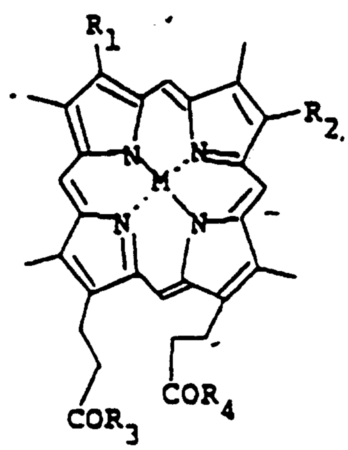

dans laquelle

$R_1$ et $R_2$ sont chacun $-CH(OR)CH_3$;
$R_3$ et $R_4$ sont un résidu obtenu en retirant un atome d'hydrogène d'un acide aspartique;
R est un alkyle, un alcényle ou un perfluoroalkyle ayant moins de 20 atomes de carbone ou un composé cyclique ayant moins de sept chaînons dans le noyau; et
M est Ga, In, Zn, Mn ou Fe.

2. Composé métalloporphyrine de formule générale (I) selon la revendication 1, pour une utilisation en tant qu'agent thérapeutique ou diagnostique pour des tumeurs malignes, en tant que marqueur de tumeur ou en tant qu'agent pour une utilisation dans la thérapeutique ciblée de cancers.

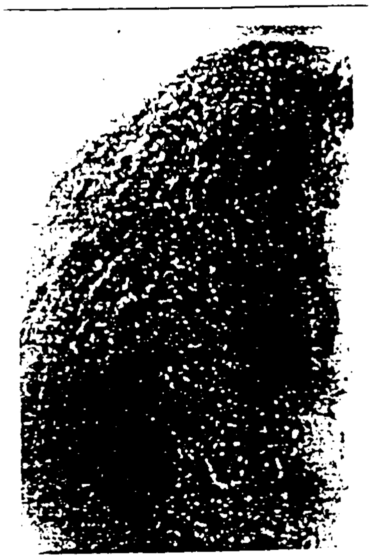

Fig. 4

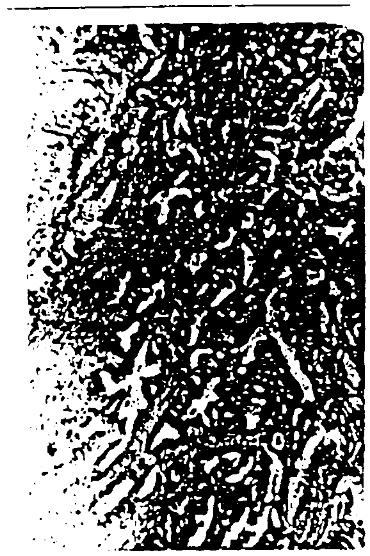

Fig. 5

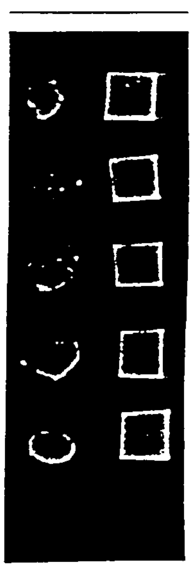

Fig. 1

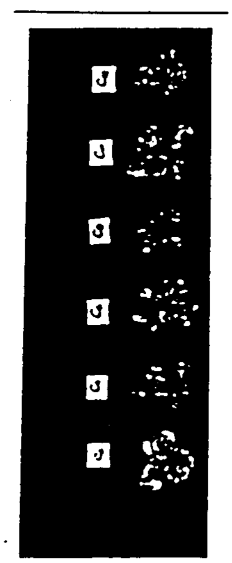

Fig. 2

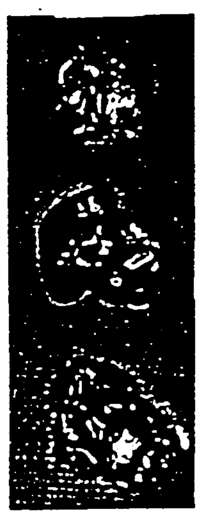

Fig. 3

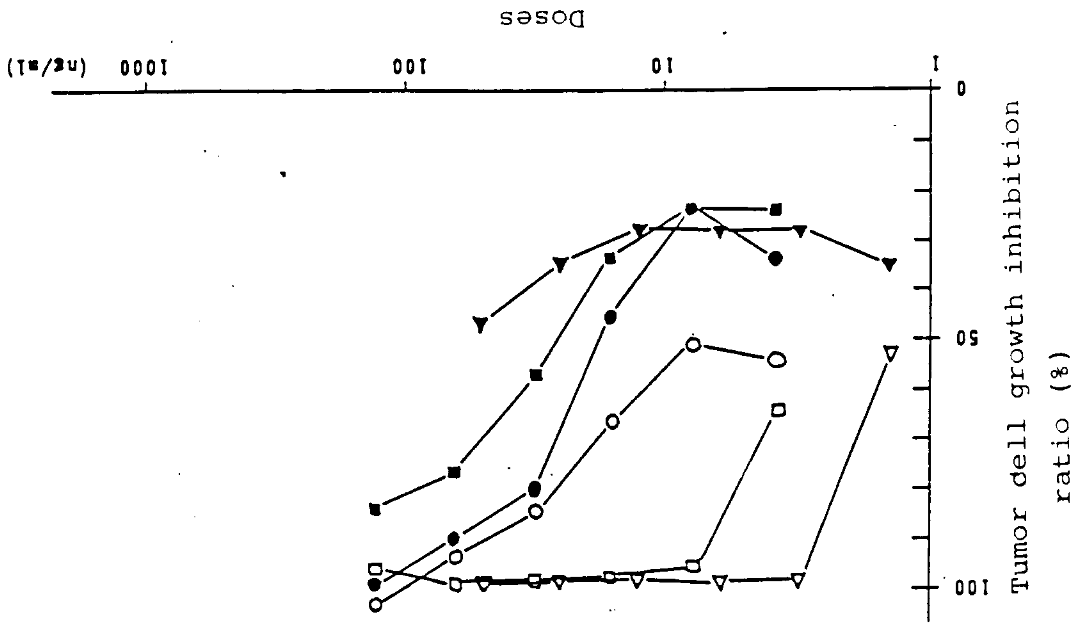
Doses
(ng/ml)
1000
100
10
1
0
50
100
Tumor dell growth inhibition ratio (%)

Fig. 7

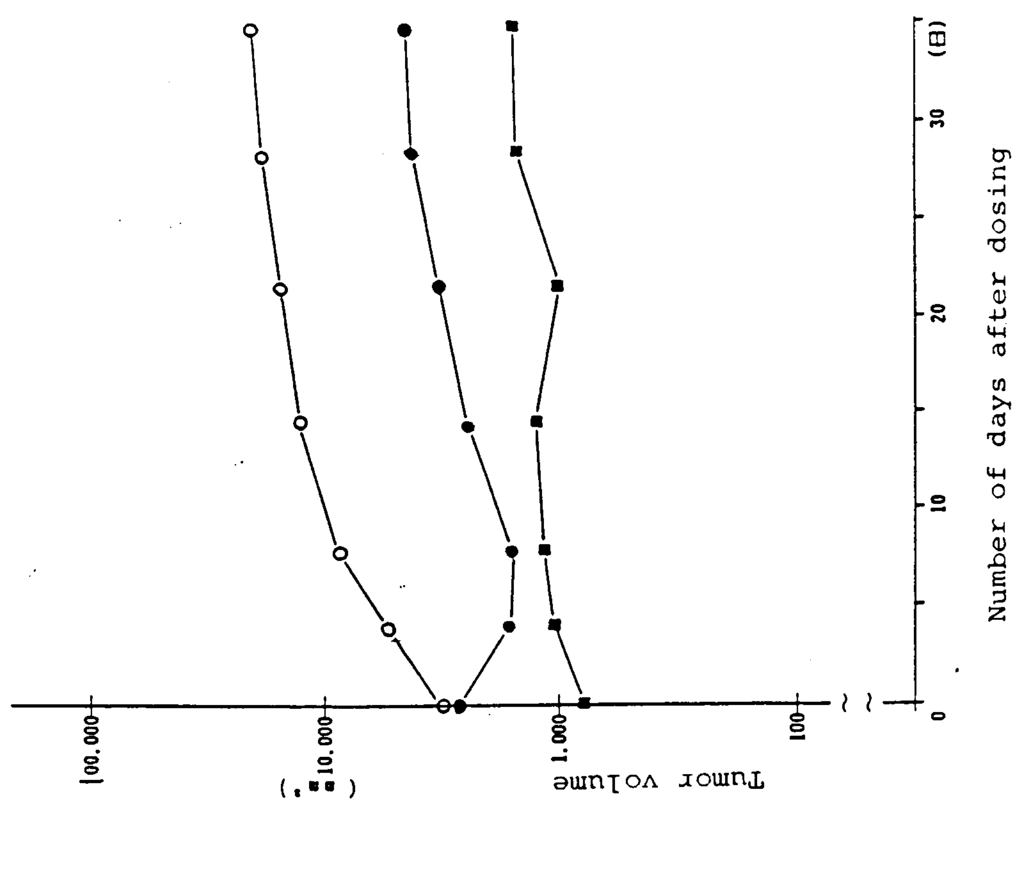
Tumor volume (mm³)
100.000
10.000
1.000
100
0
10
20
30
(日)
Number of days after dosing

Fig. 6

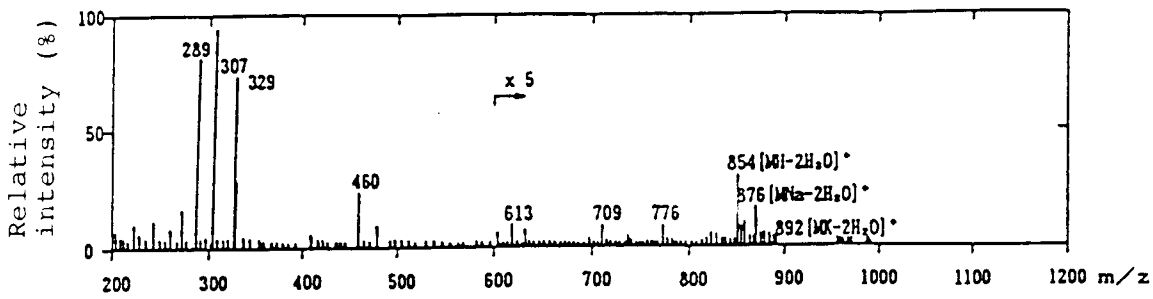


Fig. 10

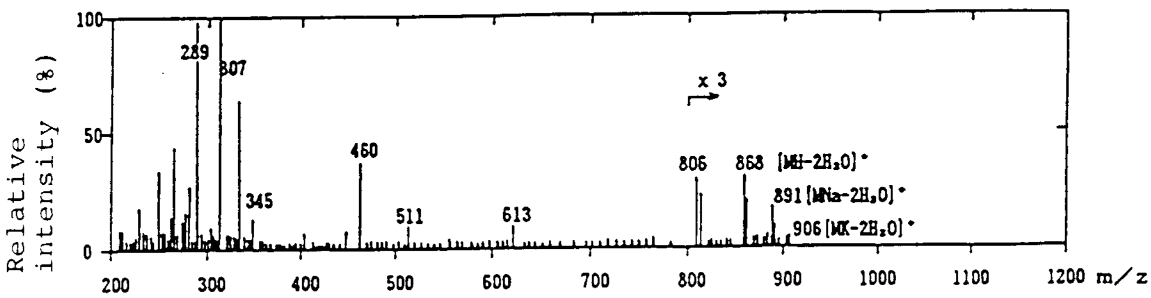


Fig. 11

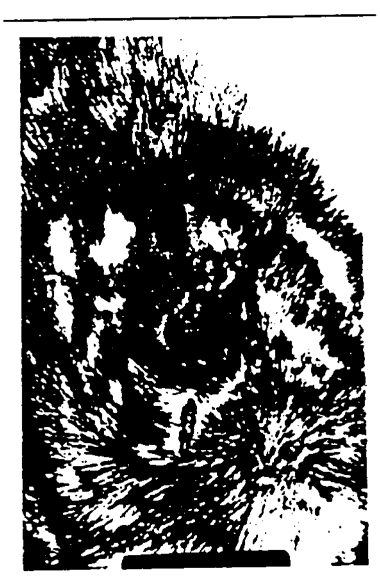

Fig. 8

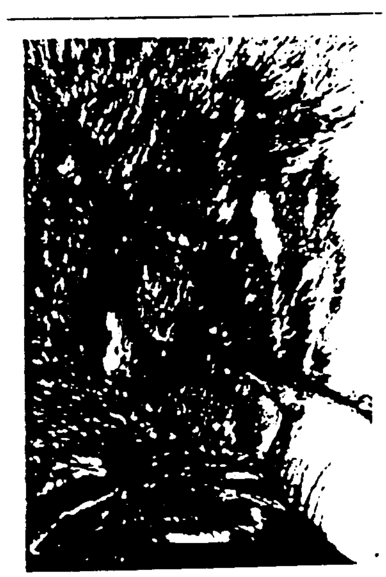

Fig. 9

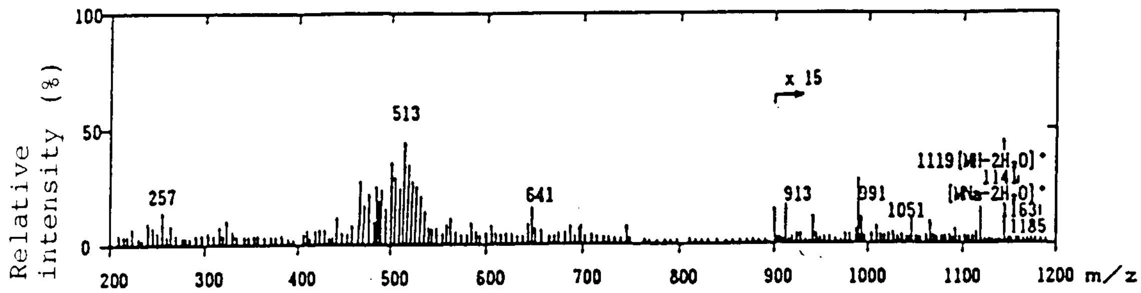


Fig. 14

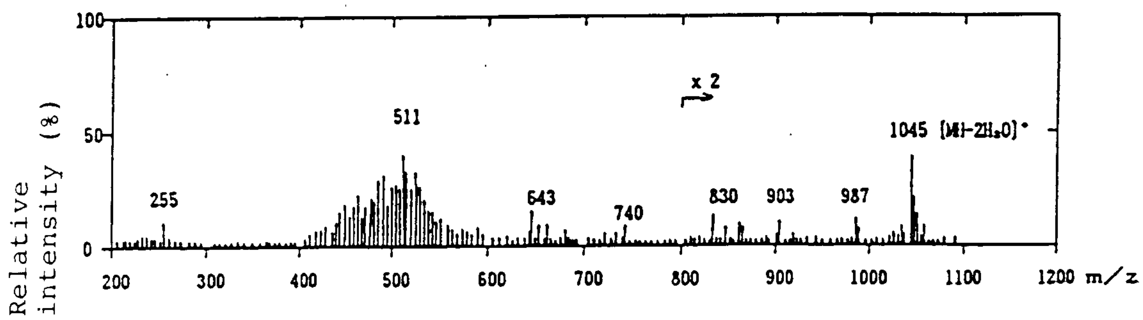


Fig. 15

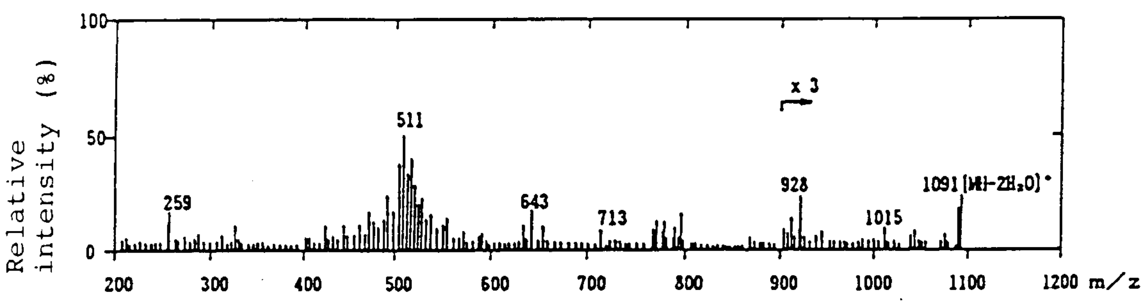


Fig. 12

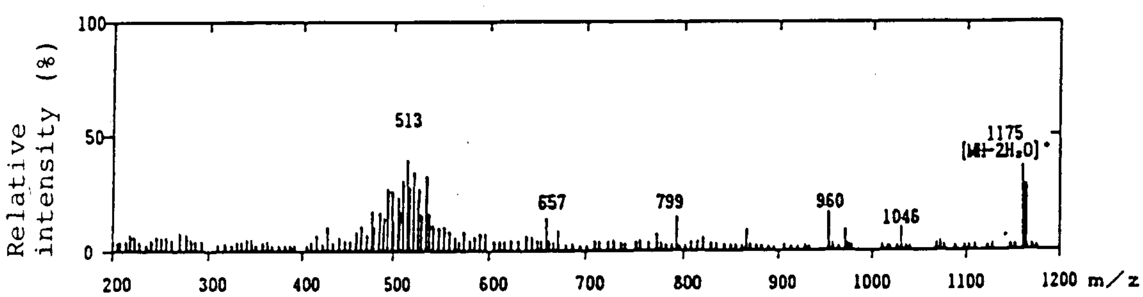


Fig. 13

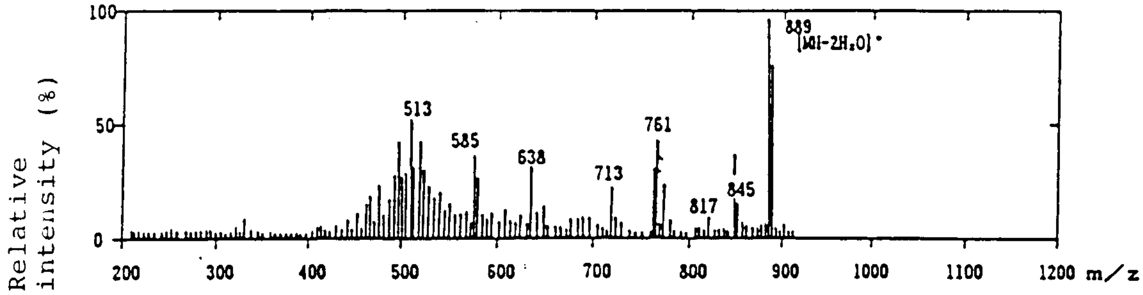


Fig. 18

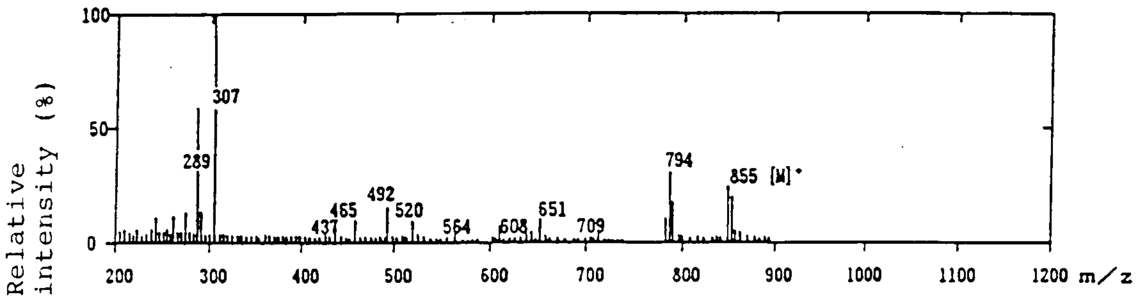


Fig. 19

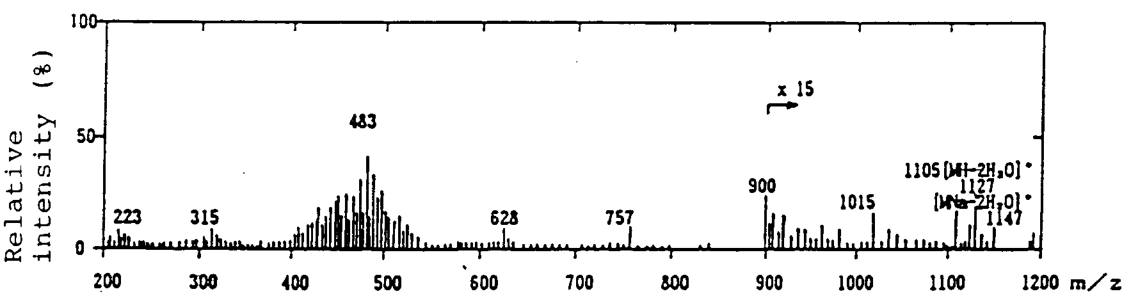


Fig. 16

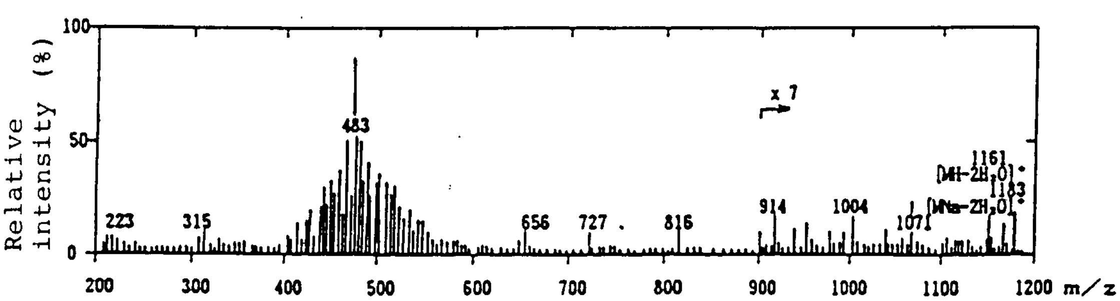


Fig. 17

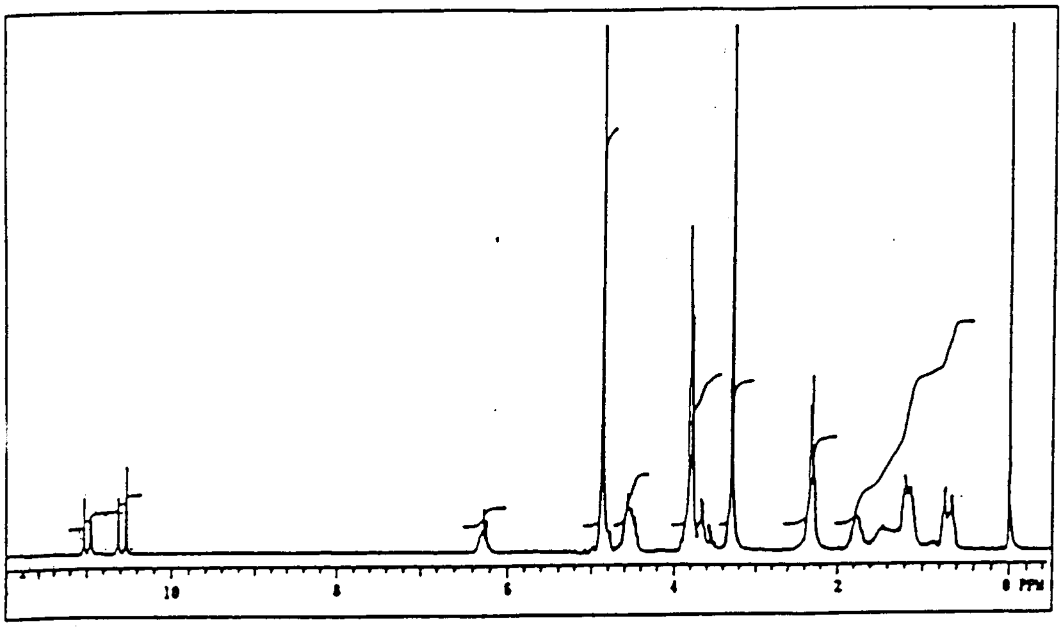


Fig. 21

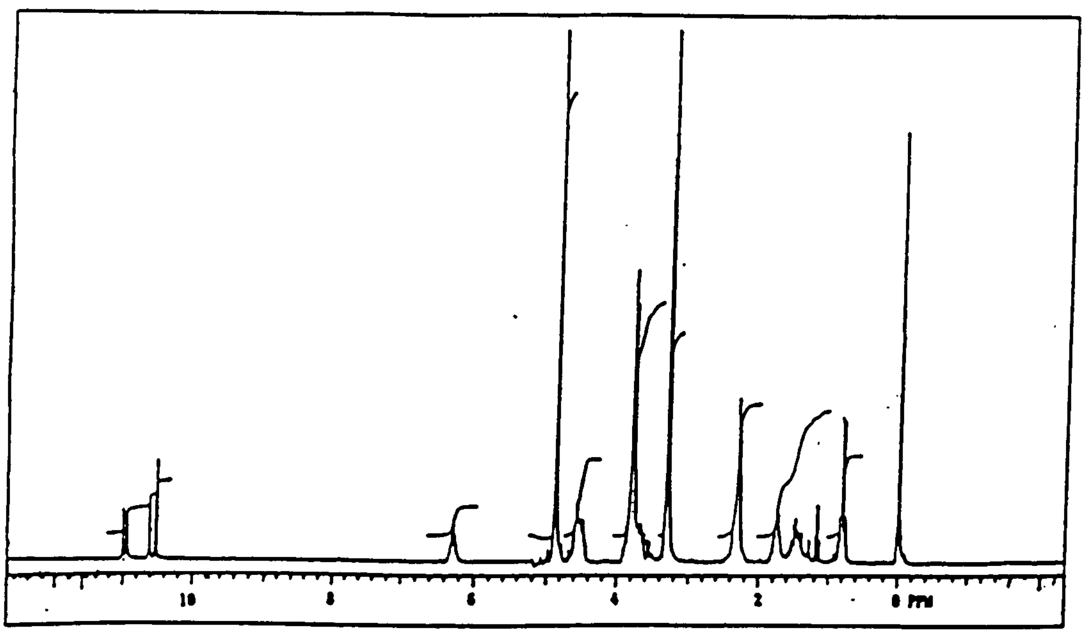


Fig. 20

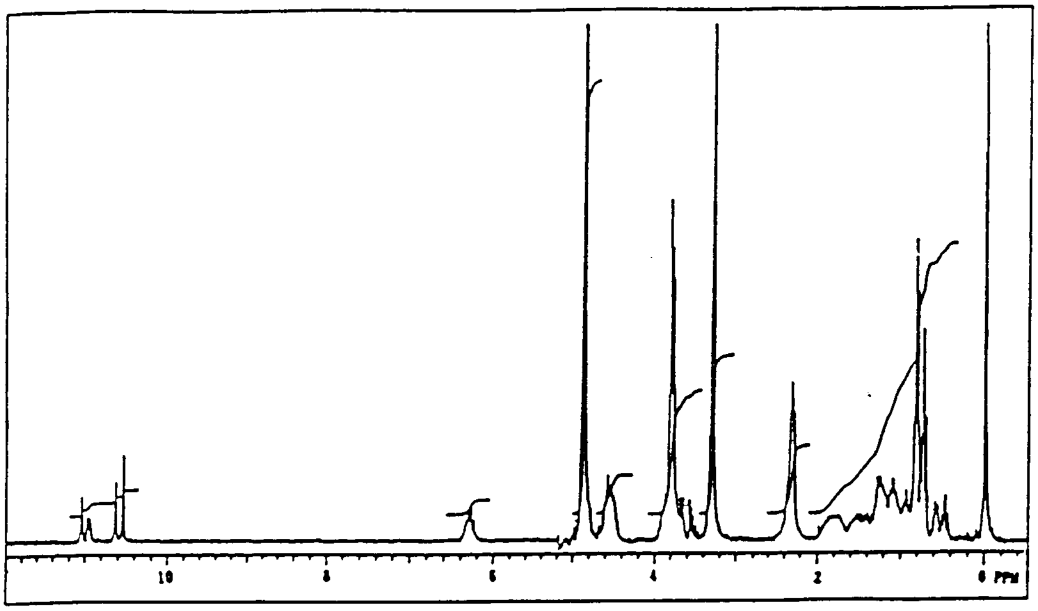

Fig. 23

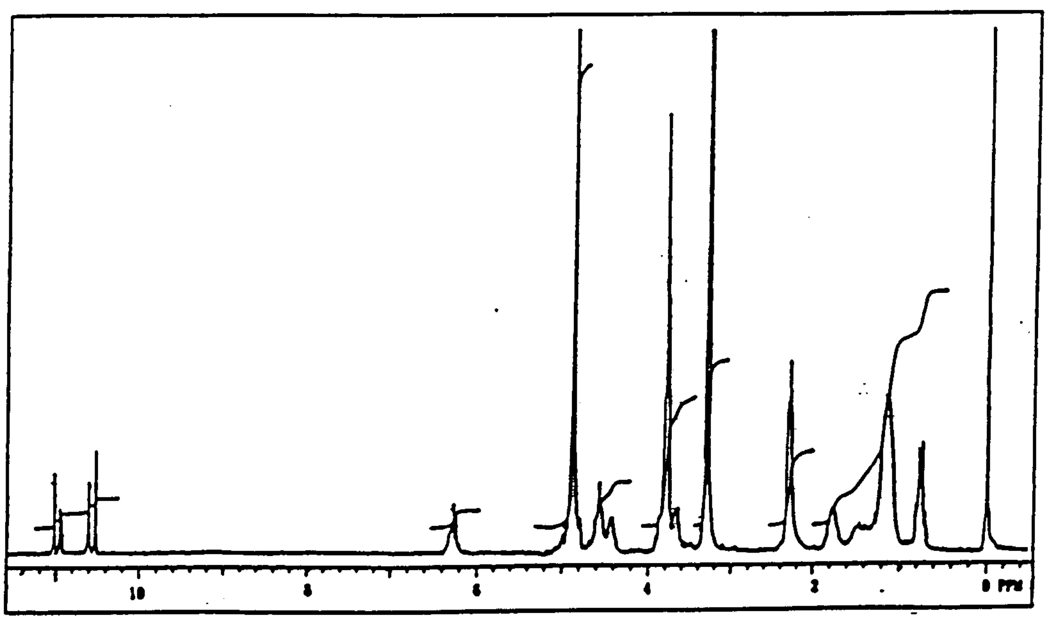

Fig. 22

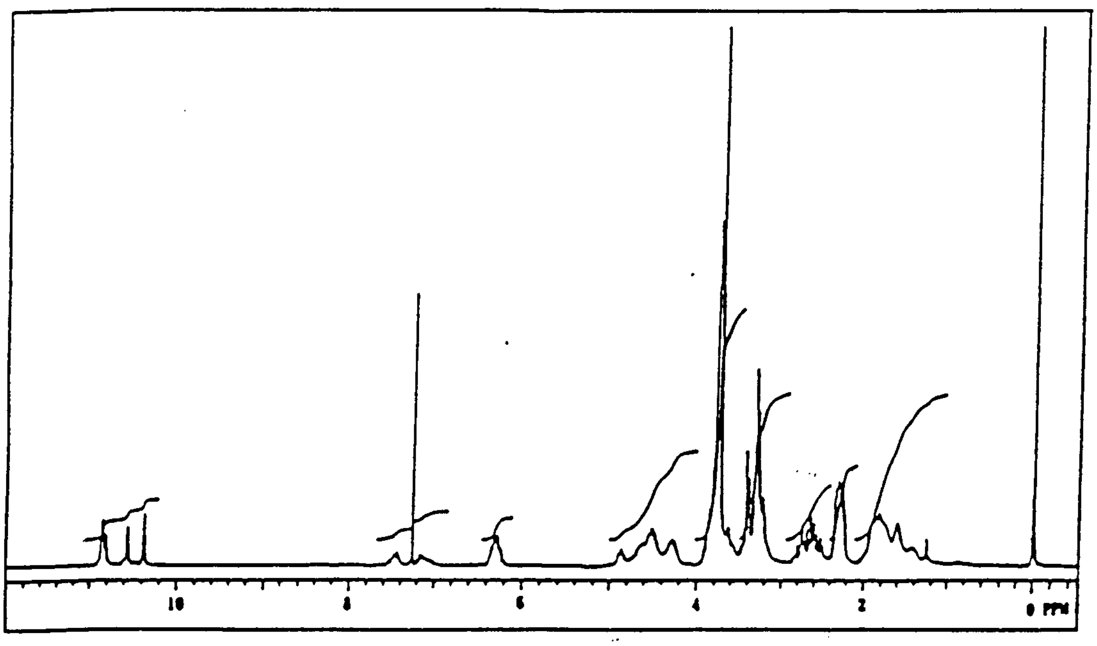


Fig. 24